# EUROPEAN PATENT APPLICATION

(11) **EP 1 561 810 A2**
(43) Date of publication of application: **10.08.2005**
(21) Application number: 05001359.8
(22) Date of filing: 24.01.2005
(51) Int. Cl.: C12N 1/20, C12N 1/21, C12N 1/00, C12N 15/03, C12P 21/00

(54) **Mutant microorganism and method for producing peptide using the same**

(30) Priority: 05.02.2004 JP 2004029844
(71) Applicant: Ajinomoto Co., Inc., Tokyo (JP)
(72) Inventor: Kira, Ikuo, Kawasaki-shi Kanagawa 210-8681 (JP); Yokozeki, Kenzo, Kawasaki-shi Kanagawa 210-8681 (JP); Suzuki, Sonoko, Kawasaki-shi Kanagawa 210-8681 (JP); Mihara, Yasuhiro, Kawasaki-shi Kanagawa 210-8681 (JP); Hirao, Yoshinori, Kawasaki-shi Kanagawa 210-8681 (JP)
(74) Representative: Strehl Schübel-Hopf & Partner

(57) **Abstract**

The present invention provides a method for producing the peptides comprising:
cultivating in medium the microorganisms wherein at least one gene selected from the group consisting of a gene encoding; aminoacylhistidine peptidase; a gene encoding leucylaminopeptidase; and a gene encoding isoaspartyldipeptidase, respectively; has been disrupted on the chromosome and wherein preferably transformed with the recombinant DNA, comprising polynucleotide encoding the proteins having peptide-synthesizing activity,
mixing at least one of the cultivated microorganisms and the disrupted cells of the microorganisms with the carboxy and amine components for the peptide synthesis.

SEQUENCE LISTING

<110> Ajinomoto Co., Inc.

<120> MUTANT MICROORGANISM AND METHOD FOR PRODUCING PEPTIDE USING THE SAME

<130> EPA-63618

<160> 22

<170> PatentIn version 3.1

<210> 1
<211> 20
<212> DNA
<213> Artificial

<150>
<151> Inventor: KIRA, Ikuo
Inventor: YOKOZEKI, Kenzo
Inventor: SUZUKI, Sonoko
Inventor: MIHARA, Yasuhiro
Inventor: HIRAO, Yoshinori

<220>
<223> PCR Primer

<400> 1
gatctggcgc actaaaaacc 20

<210> 2
<211> 20
<212> DNA
<213> Artificial

<220>
<223>  PCR Primer

<400>  2
gggatggctt ttatcgaagg                                                    20

<210>  3
<211>  20
<212>  DNA
<213>  Artificial

<220>
<223>  PCR primer

<400>  3
caaggagtta ccatgattga                                                    20

<210>  4
<211>  20
<212>  DNA
<213>  Artificial

<220>
<223>  PCR Primer

<400>  4
aaccgtttaa gccgtttcaa                                                    20

<210>  5
<211>  20
<212>  DNA
<213>  Artificial

<220>
<223>  PCR Primer

<400>  5
tatttgttat ttccattggc                                                    20

<210>  6
<211>  20
<212>  DNA
<213>  Artificial

<220>
<223>  PCR Primer

<400>  6
aatgtcgctc aaccttgaac                                                    20

<210>  7
<211>  20
<212>  DNA
<213>  Artificial

<220>
<223>  PCR Primer

<400>  7
acagcggaca tgagttacga                                                    20

<210>  8
<211>  20
<212>  DNA
<213>  Artificial

<220>
<223>  PCR Primer

<400>  8
cccgctaaat tatgcggaac                                                    20

<210> 9
<211> 9
<212> PRT
<213> Empedobacter brevis

<400> 9
Leu Phe Thr Ala Ile Tyr Gln Pro Lys
  1               5

<210> 10
<211> 9
<212> PRT
<213> Empedobacter brevis

<400> 10
Thr Asn Val Thr Tyr Thr Met Pro Asp
  1               5

<210> 11
<211> 20
<212> DNA
<213> Artificial Sequence

<220>
<223> Description of Artificial Sequence:Mix primer

<400> 11

ttyacngcna thtaycarcc                                                20

<210> 12
<211> 23
<212> DNA
<213> Artificial Sequence

<220>
<223> Description of Artificial Sequence:Mix Primer

<400> 12
tcnggcatng trtangtnac rtt                                             23

<210> 13
<211> 2024
<212> DNA
<213> Empedobacter brevis

<220>
<221> CDS
<222> (61)..(1908)
<223> gene coding  peptide-forming enzyme

<400> 13
atttcttaat aaaaactgaa atcttaatac atttatacta tcgtaaaatt tattgaacac 60

gtg aaa aaa tta aca tta aaa gta act cta ctt aca ctt ttg ttg gga   108
Val Lys Lys Leu Thr Leu Lys Val Thr Leu Leu Thr Leu Leu Leu Gly
  1               5                  10                  15

agt aca gtt gga ttt gcg caa gat gca aaa gca gat tct gct tat gtg   156
Ser Thr Val Gly Phe Ala Gln Asp Ala Lys Ala Asp Ser Ala Tyr Val
             20                  25                  30

cgc gac aat tac gaa aaa ata gaa caa gta att ccg atg cgc gat ggt 204
Arg Asp Asn Tyr Glu Lys Ile Glu Gln Val Ile Pro Met Arg Asp Gly
        35                  40                  45

aca aag tta ttt aca gct att tat cag cca aaa gat aaa aca aaa caa 252
Thr Lys Leu Phe Thr Ala Ile Tyr Gln Pro Lys Asp Lys Thr Lys Gln
    50                  55                  60

tat ccc gtt ttg tta aat cgt acg cct tat aca gtt gcg cct tat ggt 300
Tyr Pro Val Leu Leu Asn Arg Thr Pro Tyr Thr Val Ala Pro Tyr Gly
65                  70                  75                  80

gta aat gaa tac aag aaa tcg tta gga aat ttt cct aca gaa atg cgc 348
Val Asn Glu Tyr Lys Lys Ser Leu Gly Asn Phe Pro Thr Glu Met Arg
                85                  90                  95

gaa ggt ttt att ttt gtt tac caa gat gtg aga gga aaa tgg atg agc 396
Glu Gly Phe Ile Phe Val Tyr Gln Asp Val Arg Gly Lys Trp Met Ser
            100                 105                 110

gaa ggc gaa ttt gaa gat gtt cga cct ata aat cct tca aaa agt aaa 444
Glu Gly Glu Phe Glu Asp Val Arg Pro Ile Asn Pro Ser Lys Ser Lys
        115                 120                 125

aag gca att gac gaa agc aca gat aca ttt gat acg cta gaa tgg ctt 492
Lys Ala Ile Asp Glu Ser Thr Asp Thr Phe Asp Thr Leu Glu Trp Leu
    130                 135                 140

gct aaa aac ttg aag aat tac acg aaa aaa gct gga att tat gga att 540
Ala Lys Asn Leu Lys Asn Tyr Thr Lys Lys Ala Gly Ile Tyr Gly Ile
145                 150                 155                 160

tcg tat cct ggt ttt tat tcg aca atg agt ttg gtt aat tcg cat cca 588
Ser Tyr Pro Gly Phe Tyr Ser Thr Met Ser Leu Val Asn Ser His Pro
                165                 170                 175

act cta aaa gcc gtt tcg cca caa gcg ccc gtt acc aat tgg ttt tta 636
Thr Leu Lys Ala Val Ser Pro Gln Ala Pro Val Thr Asn Trp Phe Leu
            180                 185                 190

ggt gac gat ttt cat cat aat gga gtt tta ttc ttg aat gat tct ttc 684
Gly Asp Asp Phe His His Asn Gly Val Leu Phe Leu Asn Asp Ser Phe
        195                 200                 205

tca ttt atg act ttt ttt ggt gta aaa cgt ccg caa cca att acg cca 732
Ser Phe Met Thr Phe Phe Gly Val Lys Arg Pro Gln Pro Ile Thr Pro
    210                 215                 220

gat aaa ggt ccg aaa cgt ttt gaa tat cca ata aaa gat aat tat aga 780
Asp Lys Gly Pro Lys Arg Phe Glu Tyr Pro Ile Lys Asp Asn Tyr Arg
225                 230                 235                 240

ttt tat gca agt ggc tct gta aaa gag ttg aaa gat aaa tat ttg caa 828
Phe Tyr Ala Ser Gly Ser Val Lys Glu Leu Lys Asp Lys Tyr Leu Gln
                245                 250                 255

gat aat atc aag ttt tac aat gat tta ttt gcg cat cca gat tac gat 876
Asp Asn Ile Lys Phe Tyr Asn Asp Leu Phe Ala His Pro Asp Tyr Asp
            260                 265                 270

caa ttt tgg caa gat cgt aat gtt tta cca cat tta act aac gtg caa 924
Gln Phe Trp Gln Asp Arg Asn Val Leu Pro His Leu Thr Asn Val Gln
        275                 280                 285

cct gct gta atg acg gtt gga ggt ttt ttt gat gca gaa gat gtc tac 972
Pro Ala Val Met Thr Val Gly Gly Phe Phe Asp Ala Glu Asp Val Tyr
    290                 295                 300

ggc gct ttc gaa acg tat aaa gca att gag aaa caa aat ccg aaa gca 1020
Gly Ala Phe Glu Thr Tyr Lys Ala Ile Glu Lys Gln Asn Pro Lys Ala
305                 310                 315                 320

aca aat att atg gtt gcc gga cct tgg ttt cat ggt ggt tgg gtt cgt 1068
Thr Asn Ile Met Val Ala Gly Pro Trp Phe His Gly Gly Trp Val Arg
                325                 330                 335

agc aac gga agt act ttt gga gat atg caa ttt gca tcg aat aca agt 1116
Ser Asn Gly Ser Thr Phe Gly Asp Met Gln Phe Ala Ser Asn Thr Ser
            340                 345                 350

gag cat tat cag caa gaa ata gaa ttg cct ttt ttt aat tat tac tta 1164
Glu His Tyr Gln Gln Glu Ile Glu Leu Pro Phe Phe Asn Tyr Tyr Leu
        355                 360                 365

aaa gat aaa ggt aat ttt aaa cca acc gaa gct aca att ttt att acg 1212
Lys Asp Lys Gly Asn Phe Lys Pro Thr Glu Ala Thr Ile Phe Ile Thr
    370                 375                 380

gga tct aac gaa tgg aaa caa ttt gat gct tgg cca cca aaa aat gta 1260
Gly Ser Asn Glu Trp Lys Gln Phe Asp Ala Trp Pro Pro Lys Asn Val
385                 390                 395                 400

aca aca caa aaa att tat ttg caa caa aat ggt aaa ata gct ttt aat 1308
Thr Thr Gln Lys Ile Tyr Leu Gln Gln Asn Gly Lys Ile Ala Phe Asn
                405                 410                 415

aaa acc aat aca aca act act ttt gac gaa tat gtt gca gat cca aat 1356
Lys Thr Asn Thr Thr Thr Thr Phe Asp Glu Tyr Val Ala Asp Pro Asn
            420                 425                 430

tct cca gtt cct tat tca gga gga gtt tta gaa act cgt tca aga gaa 1404
Ser Pro Val Pro Tyr Ser Gly Gly Val Leu Glu Thr Arg Ser Arg Glu
        435                 440                 445

tat atg gtc gat gat caa cgc ttt gct tct act cgt cct gat gtt atg 1452
Tyr Met Val Asp Asp Gln Arg Phe Ala Ser Thr Arg Pro Asp Val Met
    450                 455                 460

gtg tat caa tct gat att ttg aca gaa gat att acg ctt gct ggt cct 1500
Val Tyr Gln Ser Asp Ile Leu Thr Glu Asp Ile Thr Leu Ala Gly Pro
465                 470                 475                 480

gtt atc aat cat tta gtg gtt tct act acg gga aca gac gct gat tat 1548
Val Ile Asn His Leu Val Val Ser Thr Thr Gly Thr Asp Ala Asp Tyr
                485                 490                 495

gtt gta aaa ttg att gat gtt tat cct gaa aac acg cca aaa ttt aat 1596
Val Val Lys Leu Ile Asp Val Tyr Pro Glu Asn Thr Pro Lys Phe Asn
            500                 505                 510

aac aaa tta atg gct gga tat caa aat ttg att cgt gca gaa att atg 1644
Asn Lys Leu Met Ala Gly Tyr Gln Asn Leu Ile Arg Ala Glu Ile Met
        515                 520                 525

cgc gga aaa tat aga aat agt ttc tct aac ccc gaa gct atg gtt ccg 1692
Arg Gly Lys Tyr Arg Asn Ser Phe Ser Asn Pro Glu Ala Met Val Pro
    530                 535                 540

aat aaa gaa aca aat gta acg tac acg atg cca gat gtt gga cat aca 1740
Asn Lys Glu Thr Asn Val Thr Tyr Thr Met Pro Asp Val Gly His Thr
545                 550                 555                 560

ttt aag aaa gga cat cgc att atg att caa gtt cag aac agt tgg ttt 1788
Phe Lys Lys Gly His Arg Ile Met Ile Gln Val Gln Asn Ser Trp Phe
                565                 570                 575

cct tta gca gat cgc aat ccg caa caa ttt atg aat gtt tac gaa gca 1836
Pro Leu Ala Asp Arg Asn Pro Gln Gln Phe Met Asn Val Tyr Glu Ala
            580                 585                 590

act tct aaa gat tat tta aaa caa acg caa cga att tat cat act tct 1884
Thr Ser Lys Asp Tyr Leu Lys Gln Thr Gln Arg Ile Tyr His Thr Ser
        595                 600                 605

tat atc gaa att ccg gta ttg aaa taacaaaaaa atccagctaa ttagctggat 1938
Tyr Ile Glu Ile Pro Val Leu Lys
    610                 615

tttttttata atgttacttt tcctattttt cctttatttc caactaaaat tacatatttt 1998

ttatcgggcg aaaccgtaca agtatg 2024

<210> 14
<211> 616
<212> PRT
<213> Empedobacter brevis

<400> 14
Val Lys Lys Leu Thr Leu Lys Val Thr Leu Leu Thr Leu Leu Leu Gly
1               5                   10                  15

Ser Thr Val Gly Phe Ala Gln Asp Ala Lys Ala Asp Ser Ala Tyr Val
            20                  25                  30

Arg Asp Asn Tyr Glu Lys Ile Glu Gln Val Ile Pro Met Arg Asp Gly
        35                  40                  45

Thr Lys Leu Phe Thr Ala Ile Tyr Gln Pro Lys Asp Lys Thr Lys Gln
    50                  55                  60

Tyr Pro Val Leu Leu Asn Arg Thr Pro Tyr Thr Val Ala Pro Tyr Gly
65                  70                  75                  80

Val Asn Glu Tyr Lys Lys Ser Leu Gly Asn Phe Pro Thr Glu Met Arg
                85                  90                  95

Glu Gly Phe Ile Phe Val Tyr Gln Asp Val Arg Gly Lys Trp Met Ser
            100                 105                 110

Glu Gly Glu Phe Glu Asp Val Arg Pro Ile Asn Pro Ser Lys Ser Lys
        115                 120                 125

Lys Ala Ile Asp Glu Ser Thr Asp Thr Phe Asp Thr Leu Glu Trp Leu
    130                 135                 140

Ala Lys Asn Leu Lys Asn Tyr Thr Lys Lys Ala Gly Ile Tyr Gly Ile
145                 150                 155                 160

Ser Tyr Pro Gly Phe Tyr Ser Thr Met Ser Leu Val Asn Ser His Pro
                165                 170                 175

Thr Leu Lys Ala Val Ser Pro Gln Ala Pro Val Thr Asn Trp Phe Leu
            180                 185                 190

Gly Asp Asp Phe His His Asn Gly Val Leu Phe Leu Asn Asp Ser Phe
        195                 200                 205

Ser Phe Met Thr Phe Phe Gly Val Lys Arg Pro Gln Pro Ile Thr Pro
    210                 215                 220

Asp Lys Gly Pro Lys Arg Phe Glu Tyr Pro Ile Lys Asp Asn Tyr Arg
225                 230                 235                 240

Phe Tyr Ala Ser Gly Ser Val Lys Glu Leu Lys Asp Lys Tyr Leu Gln
                245                 250                 255

Asp Asn Ile Lys Phe Tyr Asn Asp Leu Phe Ala His Pro Asp Tyr Asp
            260                 265                 270

Gln Phe Trp Gln Asp Arg Asn Val Leu Pro His Leu Thr Asn Val Gln
        275                 280                 285

Pro Ala Val Met Thr Val Gly Gly Phe Phe Asp Ala Glu Asp Val Tyr
    290                 295                 300

Gly Ala Phe Glu Thr Tyr Lys Ala Ile Glu Lys Gln Asn Pro Lys Ala
305                 310                 315                 320

Thr Asn Ile Met Val Ala Gly Pro Trp Phe His Gly Gly Trp Val Arg
                325                 330                 335

Ser Asn Gly Ser Thr Phe Gly Asp Met Gln Phe Ala Ser Asn Thr Ser
            340                 345                 350

Glu His Tyr Gln Gln Glu Ile Glu Leu Pro Phe Phe Asn Tyr Tyr Leu
        355                 360                 365

Lys Asp Lys Gly Asn Phe Lys Pro Thr Glu Ala Thr Ile Phe Ile Thr
    370                 375                 380

Gly Ser Asn Glu Trp Lys Gln Phe Asp Ala Trp Pro Pro Lys Asn Val
385                 390                 395                 400

Thr Thr Gln Lys Ile Tyr Leu Gln Gln Asn Gly Lys Ile Ala Phe Asn
                405                 410                 415

Lys Thr Asn Thr Thr Thr Thr Phe Asp Glu Tyr Val Ala Asp Pro Asn
            420                 425                 430

Ser Pro Val Pro Tyr Ser Gly Gly Val Leu Glu Thr Arg Ser Arg Glu
        435                 440                 445

Tyr Met Val Asp Asp Gln Arg Phe Ala Ser Thr Arg Pro Asp Val Met
    450                 455                 460

Val Tyr Gln Ser Asp Ile Leu Thr Glu Asp Ile Thr Leu Ala Gly Pro
465                 470                 475                 480

Val Ile Asn His Leu Val Val Ser Thr Thr Gly Thr Asp Ala Asp Tyr
                485                 490                 495

Val Val Lys Leu Ile Asp Val Tyr Pro Glu Asn Thr Pro Lys Phe Asn
            500                 505                 510

Asn Lys Leu Met Ala Gly Tyr Gln Asn Leu Ile Arg Ala Glu Ile Met
        515                 520                 525

Arg Gly Lys Tyr Arg Asn Ser Phe Ser Asn Pro Glu Ala Met Val Pro
    530                 535                 540

Asn Lys Glu Thr Asn Val Thr Tyr Thr Met Pro Asp Val Gly His Thr
545                 550                 555                 560

Phe Lys Lys Gly His Arg Ile Met Ile Gln Val Gln Asn Ser Trp Phe
                565                 570                 575

Pro Leu Ala Asp Arg Asn Pro Gln Gln Phe Met Asn Val Tyr Glu Ala
            580                 585                 590

Thr Ser Lys Asp Tyr Leu Lys Gln Thr Gln Arg Ile Tyr His Thr Ser
        595                 600                 605

Tyr Ile Glu Ile Pro Val Leu Lys
    610                 615

<210> 15
<211> 40
<212> DNA
<213> Artificial Sequence

<220>
<223> Description of Artificial Sequence:synthesized
primer for preparation of pTrpT

<400> 15

gtatcacgag gccctagctg tggtgtcatg gtcggtgatc                    40

<210> 16
<211> 40
<212> DNA
<213> Artificial Sequence

<220>
<223> Description of Artificial Sequence:synthesized
      primer for preparation of pTrpT

<400> 16
   ttcggggatt ccatatgata ccctttttac gtgaacttgc                    40

<210> 17
<211> 38
<212> DNA
<213> Artificial Sequence

<220>
<223> Description of Artificial Sequence:synthrsized
      primer for preparation of pTrpT_Gtg2

<400> 17
gggaattcca tatgaaaaaa ttaacattaa aagtaact                    38

<210> 18
<211> 36
<212> DNA
<213> Artificial Sequence

<220>
<223> Description of Artificial Sequence:synthesized

primer for preparation of pTrpT_Gtg2

<400> 18
gggggctgca gtacttgtac ggtttcgccc gataaa                                36

<210> 19
<211> 1935
<212> DNA
<213> Sphingobacterium sp.

<220>
<221> CDS
<222> (61)..(1917)
<223> gene of peptide-forming enzyme

<400> 19
gaaaccaagt gtaaaattat aatttacacc aaagaatgta ctgaacaaat aattatctga 60

atg aaa aat aca att tcg tgc cta act tta gcg ctt tta agc gca agc   108
Met Lys Asn Thr Ile Ser Cys Leu Thr Leu Ala Leu Leu Ser Ala Ser
1               5                   10                  15

cag tta cat gct caa aca gct gcc gac tcg gct tat gtt aga gat cat   156
Gln Leu His Ala Gln Thr Ala Ala Asp Ser Ala Tyr Val Arg Asp His
            20                  25                  30

tat gaa aag acc gaa gta gca att ccc atg cga gat ggg aaa aaa tta   204
Tyr Glu Lys Thr Glu Val Ala Ile Pro Met Arg Asp Gly Lys Lys Leu
        35                  40                  45

ttt act gcg atc tac agt cca aaa gac aaa tcc aag aaa tat cca gtt   252
Phe Thr Ala Ile Tyr Ser Pro Lys Asp Lys Ser Lys Lys Tyr Pro Val
    50                  55                  60

ttg ctc aat aga acg ccc tac acg gtt tca cct tat ggg cag aac gaa   300

Leu Leu Asn Arg Thr Pro Tyr Thr Val Ser Pro Tyr Gly Gln Asn Glu
65                  70                  75                  80

tat aaa aaa agc ttg gga aac ttt ccc caa atg atg cgt gaa ggc tat     348
Tyr Lys Lys Ser Leu Gly Asn Phe Pro Gln Met Met Arg Glu Gly Tyr
                85                  90                  95

att ttc gtt tac cag gat gtc cgt ggc aag tgg atg agc gaa ggt gat     396
Ile Phe Val Tyr Gln Asp Val Arg Gly Lys Trp Met Ser Glu Gly Asp
            100                 105                 110

ttt gaa gat ata cgt ccg acc acg tac agc aaa gat aaa aaa gca atc     444
Phe Glu Asp Ile Arg Pro Thr Thr Tyr Ser Lys Asp Lys Lys Ala Ile
        115                 120                 125

gat gaa agt acg gat acc tat gat gcg ctt gaa tgg tta cag aaa aat     492
Asp Glu Ser Thr Asp Thr Tyr Asp Ala Leu Glu Trp Leu Gln Lys Asn
    130                 135                 140

ctc aaa aac tat aat ggc aaa gcc ggg ctc tat ggg att tcc tat cca     540
Leu Lys Asn Tyr Asn Gly Lys Ala Gly Leu Tyr Gly Ile Ser Tyr Pro
145                 150                 155                 160

ggc ttc tat tct acc gtc gga ttg gtc aaa aca cac ccg agc ttg aag     588
Gly Phe Tyr Ser Thr Val Gly Leu Val Lys Thr His Pro Ser Leu Lys
                165                 170                 175

gca gtc tcc cca cag gct ccc gta aca gac tgg tat atc ggc gac gac     636
Ala Val Ser Pro Gln Ala Pro Val Thr Asp Trp Tyr Ile Gly Asp Asp
            180                 185                 190

ttc cac cat aat ggc gta ttg ttt ctt cag gat gca ttt aca ttc atg     684
Phe His His Asn Gly Val Leu Phe Leu Gln Asp Ala Phe Thr Phe Met
        195                 200                 205

tca acc ttt ggt gtc cct cgt cca aaa ccc att aca ccg gat caa ttt     732

Ser Thr Phe Gly Val Pro Arg Pro Lys Pro Ile Thr Pro Asp Gln Phe
    210                 215                 220

aag ggc aaa att cag atc aaa gaa gcc gat aaa tat aac ttt ttt gca    780
Lys Gly Lys Ile Gln Ile Lys Glu Ala Asp Lys Tyr Asn Phe Phe Ala
225                 230                 235                 240

gaa gca gga aca gcg cgg gaa ctc aaa gaa aag tat ttt ggt gac tcc    828
Glu Ala Gly Thr Ala Arg Glu Leu Lys Glu Lys Tyr Phe Gly Asp Ser
                245                 250                 255

gta caa ttt tgg aat gac ctg ttt aag cat ccc gac tat gat gat ttt    876
Val Gln Phe Trp Asn Asp Leu Phe Lys His Pro Asp Tyr Asp Asp Phe
            260                 265                 270

tgg aaa tcg cgt gtg atc acg aat tct tta cag gag gta aaa cca gct    924
Trp Lys Ser Arg Val Ile Thr Asn Ser Leu Gln Glu Val Lys Pro Ala
        275                 280                 285

gtg atg gtg gtt ggt ggt ttc ttt gac gcg gaa gat gct tat gga aca    972
Val Met Val Val Gly Gly Phe Phe Asp Ala Glu Asp Ala Tyr Gly Thr
    290                 295                 300

ttt aag acc tac caa tcg att gag gat aaa agc aaa aaa aac aac tcg    1020
Phe Lys Thr Tyr Gln Ser Ile Glu Asp Lys Ser Lys Lys Asn Asn Ser
305                 310                 315                 320

att tta gtc gcg gga cct tgg tat cat ggc ggt tgg gtt cgt gca gaa    1068
Ile Leu Val Ala Gly Pro Trp Tyr His Gly Gly Trp Val Arg Ala Glu
                325                 330                 335

gga aac tat tta ggt gat atc caa ttt gag aaa aaa acc agt att act    1116
Gly Asn Tyr Leu Gly Asp Ile Gln Phe Glu Lys Lys Thr Ser Ile Thr
            340                 345                 350

tat cag gaa caa ttt gaa caa cca ttt ttc aaa tat tac cta aaa gat    1164

Tyr Gln Glu Gln Phe Glu Gln Pro Phe Phe Lys Tyr Tyr Leu Lys Asp
        355                 360                 365

gaa gga aac ttc gcc cct tcc gaa gct aac att ttt gtt tca ggc agc 1212
Glu Gly Asn Phe Ala Pro Ser Glu Ala Asn Ile Phe Val Ser Gly Ser
    370                 375                 380

aac gaa tgg aaa cat ttc gaa cag tgg cca cca aaa aat gta gag aca 1260
Asn Glu Trp Lys His Phe Glu Gln Trp Pro Pro Lys Asn Val Glu Thr
385                 390                 395                 400

aaa aaa cta tac ttc caa cct cag ggg aaa ctt gga ttt gac aaa gtt 1308
Lys Lys Leu Tyr Phe Gln Pro Gln Gly Lys Leu Gly Phe Asp Lys Val
                405                 410                 415

caa cgt aca gat tcc tgg gat gaa tat gta aca gac cct aat aaa cct 1356
Gln Arg Thr Asp Ser Trp Asp Glu Tyr Val Thr Asp Pro Asn Lys Pro
            420                 425                 430

gtt ccg cat caa ggt ggg gta att caa aac cga aca cgg gag tat atg 1404
Val Pro His Gln Gly Gly Val Ile Gln Asn Arg Thr Arg Glu Tyr Met
        435                 440                 445

gta gat gat caa cgt ttc gcg gct agt cgc cct gat gtc atg gtt tat 1452
Val Asp Asp Gln Arg Phe Ala Ala Ser Arg Pro Asp Val Met Val Tyr
    450                 455                 460

caa acg gaa ccg ttg acg gag gac ctg acg ata gta ggc cca atc aaa 1500
Gln Thr Glu Pro Leu Thr Glu Asp Leu Thr Ile Val Gly Pro Ile Lys
465                 470                 475                 480

aac ttt ctc aaa gtt tct tca aca gga aca gac gcg gac tat gtt gtc 1548
Asn Phe Leu Lys Val Ser Ser Thr Gly Thr Asp Ala Asp Tyr Val Val
                485                 490                 495

aaa ctg att gac gtt tat ccg aat gat gca gca agt tat caa gga aaa 1596

Lys Leu Ile Asp Val Tyr Pro Asn Asp Ala Ala Ser Tyr Gln Gly Lys
            500                 505                 510

aca atg gct gga tat caa atg atg gta cgt ggt gag atc atg gcg ggg 1644
Thr Met Ala Gly Tyr Gln Met Met Val Arg Gly Glu Ile Met Ala Gly
        515                 520                 525

aaa tac cga aat ggt ttc gat aaa gcg cag gcc ttg act cca ggt atg 1692
Lys Tyr Arg Asn Gly Phe Asp Lys Ala Gln Ala Leu Thr Pro Gly Met
    530                 535                 540

gtc gaa aag gtg aat ttt gaa atg cca gac gtt gcg cat acc ttc aaa 1740
Val Glu Lys Val Asn Phe Glu Met Pro Asp Val Ala His Thr Phe Lys
545                 550                 555                 560

aaa gga cat cgc att atg gtt cag gta caa aac tca tgg ttt ccg ctg 1788
Lys Gly His Arg Ile Met Val Gln Val Gln Asn Ser Trp Phe Pro Leu
                565                 570                 575

gca gaa cga aat cca cag gtg ttt tta gca cct tat aca gct acc aaa 1836
Ala Glu Arg Asn Pro Gln Val Phe Leu Ala Pro Tyr Thr Ala Thr Lys
            580                 585                 590

gct gat ttc cgc aaa gct acc caa cgt att ttt cac gat gtg aac aat 1884
Ala Asp Phe Arg Lys Ala Thr Gln Arg Ile Phe His Asp Val Asn Asn
        595                 600                 605

gcc aca tac atc gaa ttt tct gtc ctc aaa gat tagcaggtaa attcgaaa 1935
Ala Thr Tyr Ile Glu Phe Ser Val Leu Lys Asp
    610                 615

<210> 20
<211> 619
<212> PRT
<213> Sphingobacterium sp.

<400> 20

Met Lys Asn Thr Ile Ser Cys Leu Thr Leu Ala Leu Leu Ser Ala Ser
1               5                   10                  15

Gln Leu His Ala Gln Thr Ala Ala Asp Ser Ala Tyr Val Arg Asp His
            20                  25                  30

Tyr Glu Lys Thr Glu Val Ala Ile Pro Met Arg Asp Gly Lys Lys Leu
        35                  40                  45

Phe Thr Ala Ile Tyr Ser Pro Lys Asp Lys Ser Lys Lys Tyr Pro Val
    50                  55                  60

Leu Leu Asn Arg Thr Pro Tyr Thr Val Ser Pro Tyr Gly Gln Asn Glu
65                  70                  75                  80

Tyr Lys Lys Ser Leu Gly Asn Phe Pro Gln Met Met Arg Glu Gly Tyr
                85                  90                  95

Ile Phe Val Tyr Gln Asp Val Arg Gly Lys Trp Met Ser Glu Gly Asp
            100                 105                 110

Phe Glu Asp Ile Arg Pro Thr Thr Tyr Ser Lys Asp Lys Lys Ala Ile
        115                 120                 125

Asp Glu Ser Thr Asp Thr Tyr Asp Ala Leu Glu Trp Leu Gln Lys Asn
    130                 135                 140

Leu Lys Asn Tyr Asn Gly Lys Ala Gly Leu Tyr Gly Ile Ser Tyr Pro
145                 150                 155                 160

Gly Phe Tyr Ser Thr Val Gly Leu Val Lys Thr His Pro Ser Leu Lys
                165                 170                 175

Ala Val Ser Pro Gln Ala Pro Val Thr Asp Trp Tyr Ile Gly Asp Asp

            180                 185                 190

Phe His His Asn Gly Val Leu Phe Leu Gln Asp Ala Phe Thr Phe Met
        195                 200                 205

Ser Thr Phe Gly Val Pro Arg Pro Lys Pro Ile Thr Pro Asp Gln Phe
    210                 215                 220

Lys Gly Lys Ile Gln Ile Lys Glu Ala Asp Lys Tyr Asn Phe Phe Ala
225                 230                 235                 240

Glu Ala Gly Thr Ala Arg Glu Leu Lys Glu Lys Tyr Phe Gly Asp Ser
                245                 250                 255

Val Gln Phe Trp Asn Asp Leu Phe Lys His Pro Asp Tyr Asp Asp Phe
            260                 265                 270

Trp Lys Ser Arg Val Ile Thr Asn Ser Leu Gln Glu Val Lys Pro Ala
        275                 280                 285

Val Met Val Val Gly Gly Phe Phe Asp Ala Glu Asp Ala Tyr Gly Thr
    290                 295                 300

Phe Lys Thr Tyr Gln Ser Ile Glu Asp Lys Ser Lys Lys Asn Asn Ser
305                 310                 315                 320

Ile Leu Val Ala Gly Pro Trp Tyr His Gly Gly Trp Val Arg Ala Glu
                325                 330                 335

Gly Asn Tyr Leu Gly Asp Ile Gln Phe Glu Lys Lys Thr Ser Ile Thr
            340                 345                 350

Tyr Gln Glu Gln Phe Glu Gln Pro Phe Phe Lys Tyr Tyr Leu Lys Asp
        355                 360                 365

Glu Gly Asn Phe Ala Pro Ser Glu Ala Asn Ile Phe Val Ser Gly Ser

370                 375                 380

Asn Glu Trp Lys His Phe Glu Gln Trp Pro Pro Lys Asn Val Glu Thr
385                 390                 395                 400

Lys Lys Leu Tyr Phe Gln Pro Gln Gly Lys Leu Gly Phe Asp Lys Val
                405                 410                 415

Gln Arg Thr Asp Ser Trp Asp Glu Tyr Val Thr Asp Pro Asn Lys Pro
            420                 425                 430

Val Pro His Gln Gly Gly Val Ile Gln Asn Arg Thr Arg Glu Tyr Met
        435                 440                 445

Val Asp Asp Gln Arg Phe Ala Ala Ser Arg Pro Asp Val Met Val Tyr
    450                 455                 460

Gln Thr Glu Pro Leu Thr Glu Asp Leu Thr Ile Val Gly Pro Ile Lys
465                 470                 475                 480

Asn Phe Leu Lys Val Ser Ser Thr Gly Thr Asp Ala Asp Tyr Val Val
                485                 490                 495

Lys Leu Ile Asp Val Tyr Pro Asn Asp Ala Ala Ser Tyr Gln Gly Lys
            500                 505                 510

Thr Met Ala Gly Tyr Gln Met Met Val Arg Gly Glu Ile Met Ala Gly
        515                 520                 525

Lys Tyr Arg Asn Gly Phe Asp Lys Ala Gln Ala Leu Thr Pro Gly Met
    530                 535                 540

Val Glu Lys Val Asn Phe Glu Met Pro Asp Val Ala His Thr Phe Lys
545                 550                 555                 560

Lys Gly His Arg Ile Met Val Gln Val Gln Asn Ser Trp Phe Pro Leu

                565                 570                 575

Ala Glu Arg Asn Pro Gln Val Phe Leu Ala Pro Tyr Thr Ala Thr Lys
            580                 585                 590

Ala Asp Phe Arg Lys Ala Thr Gln Arg Ile Phe His Asp Val Asn Asn
        595                 600                 605

Ala Thr Tyr Ile Glu Phe Ser Val Leu Lys Asp
    610                 615

<210> 21
<211> 30
<212> DNA
<213> Artificial Sequence

<220>
<223> Description of Artificial Sequence:synthesized
primer for preparation of pTrpT_Sm_aet

<400> 21
gggaattcca tatgaaaaat acaatttcgt 30

<210> 22
<211> 29
<212> DNA
<213> Artificial Sequence

<220>
<223> Description of Artificial Sequence:synthesized
primer for preparation of pTrpT_Sm_aet

<400> 22

gctctagact aatctttgag gacagaaaa 29

## Description

### BACKGROUND OF THE INVENTION

### 1) Field of the Invention

The present invention relates to a mutant microorganism and a method for producing peptides using the mutant microorganism. More particularly, the present invention relates to the mutant microorganism that allow for efficient production of the peptides and the method for producing the peptides using the mutant microorganism.

### 2) Description of the Related Art

Peptides are used in various fields such as pharmaceuticals and foods. For example, L-alanyl-L-glutamine is widely used as components of solutions for transfusion and of serum-free media, because it is more stable and soluble in water than L-glutamine.

The peptides are generally produced by chemical synthetic methods. However, such methods often require complicated and inefficient steps, none of which are fully satisfactory.

On the other hand, methods for producing peptides with enzymes have also been developed. However, the conventional methods with enzymes require improvement of extremely slow rate of peptide formation, low yield of peptide formation, and so forth. Therefore, development of a novel method for producing such peptides on an industrial scale and at a high efficiency has been demanded.

The method for efficiently producing target products using microorganisms has been disclosed, which involves steps for preparing a strain disrupted of which a gene for enzyme catalyzing a reaction with the target product as a substrate, and producing the target products using them(for example, JP-A No.2003-189863).

### SUMMARY OF THE INVENTION

The method for producing the target products by preparing disrupted strains does not achieve efficient production, because its yield decreases unless enzymes, which involve in a reaction system from starting microorganisms to target products and are factors that mediate production of byproducts and decompose the target products, are appropriately disrupted. However, the reaction system including microorganisms is often complicated and thereby, it is difficult to find appropriate genes to be disrupted depending on conditions such as materials, the target products, and kinds of microorganisms to be used.

Thus, an object of the present invention is to provide a method for efficiently producing peptides using disrupted strains.

As a result of extensive studies, the inventors of the present invention have found the genes to be disrupted to achieve efficient production of peptides using microorganisms, thus accomplishing the present invention. The present invention provides the mutant microorganisms, the method for preparing them, and the method for producing peptides, all of which are in detail explained below.
1. A mutant microorganism of which at least one gene is disrupted,
   wherein the gene is selected from the group consisting of a gene encoding aminoacylhistidine peptidase, a gene encoding leucylaminopeptidase and a gene encoding isoaspartyldipeptidase, respectively on a chromosome.
2. The microorganism according to item 1, of which a gene encoding α-aspartyldipeptidase on the chromosome is disrupted.
3. The microorganism according to item 1 or 2, which is a bacterium belonging to the genus *Escherichia.*
4. A mutant microorganism:
   of which at least one gene is disrupted, wherein the gene is selected from the group consisting of a gene encoding aminoacylhistidine peptidase, a gene encoding leucylaminopeptidase and a gene encoding isoaspartyldipeptidase, respectively on a chromosome; and
   which is transformed with a recombinant DNA including a polynucleotide encoding a protein having peptide-synthesizing activity.
5. The microorganism according to item 4, of which a gene encoding α-aspartyldipeptidase on the chromosome is disrupted.
6. The microorganism according to item 4 or 5, which is a bacterium belonging to a genus *Escherichia.*
7. The microorganism according to any of items 4 to 6, wherein the protein having peptide-synthesizing activity is derived from the bacterium belonging to the genus *Empedobacter* or *Sphingobacterium.*
8. The microorganism according to any one of items 4 to 6, wherein the protein having the peptide-synthesizing activity is selected from the group consisting of (A) and (B):
   (A) a protein comprising an amino acid sequence of SEQ ID NO: 14,
   (B) a protein comprising an amino acid sequence including substitution, deletion, insertion, addition, and/or inversion of one or several amino acid residues in the amino acid sequence of SEQ ID NO: 14, and having the peptide-synthesizing activity.
9. The microorganism according to any one of items 4 to 6, wherein the protein having the peptide-synthesizing activity is selected from the group consisting of (C) or (D):
   (C) a protein comprising an amino acid sequence of SEQ ID NO: 20,
   (D) a protein comprising an amino acid sequence including substitution, deletion, insertion, addition, and/or inversion of one or several amino acid residues in the amino acid sequence of SEQ ID NO: 20, and having the peptide-synthesizing activity.
10. A method for producing a microorganism comprising:
   disrupting at least one gene selected from the group consisting of a gene encoding aminoacylhistidine peptidase, a gene encoding leucylaminopeptidase and a gene encoding isoaspartyldipeptidase, respectively on a chromosome.
11. The method for producing a microorganism according to item 10, comprising:
   disrupting a gene encoding α-aspartyldipeptidase on the chromosome.
12. A method for producing a microorganism comprising:
   disrupting at least one gene selected from the group consisting of a gene encoding aminoacylhistidine peptidase, a gene encoding leucylaminopeptidase and a gene encoding isoaspartyldipeptidase, respectively on a chromosome; and
   transforming with a recombinant DNA including a polynucleotide encoding a protein having peptide-synthesizing activity.
13. The method for producing a microorganism according to item 12, comprising:
   disrupting a gene encoding α-aspartyldipeptidase on the chromosome.
14. A method for producing a peptide comprising:
   cultivating in medium a microorganism of which at least one gene is disrupted, wherein the gene is selected from the group consisting of a gene encoding aminoacylhistidine peptidase, a gene encoding leucylaminopeptidase and a gene encoding isoaspartyldipeptidase, respectively on a chromosome, and wherein the microorganism is transformed with a recombinant DNA including a polynucleotide encoding a protein having peptide-synthesizing activity; and
   mixing at least one of the microorganism cultivated and a disrupted cell of the microorganism with a carboxy component and an amine component to form a peptide.
15. The method for producing a peptide according to item 14,
wherein a gene encoding α-aspartyldipeptidase on a chromosome of the microorganism is disrupted.

According to the present invention, the method for effectively producing the peptides may be provided. The present invention is very useful in industrial production of the peptides.

### BRIEF DESCRIPTION OF DRAWINGS

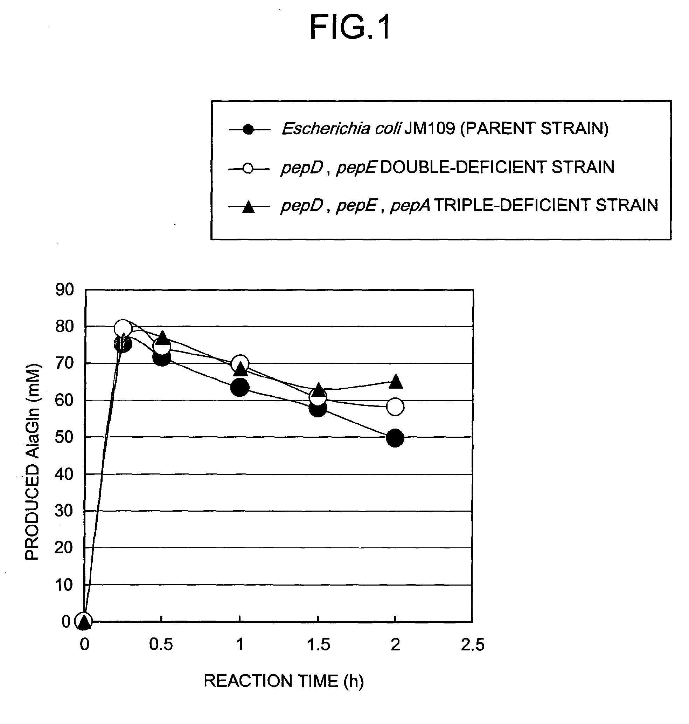

FIG.1 is a view showing the quantities of produced alanylglutamine.

### DETAILED DESCRIPTION

Embodiments are explained below referring to best modes for carrying out the present invention. Note that various gene engineering techniques described below are disclosed in many typical experimental manuals including Molecular Cloning, 2^{nd} edition, Cold Spring Harbor press (1989), Cell technology Handbook(Saibokogaku Handbook), Toshio Kuroki et al., Yodosya (1992), and New Gene Engineering Handbook(Shin-Idenshikogaku Handbook) Revision 3, edited by Muramatsu et al., Yodosya (1999). The descriptions of them are quoted in this specification.

### 1. Mutant microorganisms of the present invention and the method for producing them

The microorganisms of the present invention are produced by disrupting genes that prevent efficient formation of the peptides. The genes to be disrupted include a gene encoding aminoacyl histidinepeptidase(hereinafter, simply referred to as "*pepD* gene"), a gene encoding leucylaminopeptidase(hereinafter, "*pepA* gene"), and a gene encoding isoaspartyldipeptidase(hereinafter, "*iadA* gene"), all of which express in microorganism chromosomes. At least one of genes among these genes may be disrupted. Moreover, the microorganisms of the present invention may be exemplified as a more preferable embodiment than that of the microorganisms produced by disrupting a gene encoding α-aspartyldipeptidase(hereinafter, "*pepE* gene") on the chromosome.

The use of microorganisms produced by disrupting one or more of genes among the aforementioned gene group achieves more efficient peptide production than the use of microorganisms, in which these genes express. It is considered that the efficient production of the target peptides is achieved in this way, mainly because decomposition activity of the peptides, namely the target products, can be decreased or inhibited.

The genes referred in this specification are medium having genetic codes including, for example, polypeptides having nucleotide sequences encoding proteins.

In this specification, the disruption of a gene refers to the suppression or inhibition of the expression of gene products. More specifically, the genes may be disrupted by inducing mutation in the genes encoding peptidase having activity of decomposing peptides, namely target products, or by deleting the same genes. A specific method for disrupting genes is further explained later in this specification.

In this specification, "peptide" refers to a polymer having a peptide bond, namely a polypeptide including, for example, a dipeptide, a tripeptide, and an oligopeptide.

A preferable embodiment of the present invention includes a mode, wherein among these genes, at least a *pepD* gene is disrupted. The disruption of the *pepD* gene may significantly improve efficiency of peptide production, and is outstandingly effective, especially in the production of aspartylphenylalanine, alanylglutamine, and so forth. Further preferable embodiment includes a mode, wherein all of the *pepD, pepA, iadA*, and *pepE* genes are disrupted. When all four kinds of genes are disrupted, it is most probable that the decomposition and deletion of produced peptides can be inhibited in producing each kind of peptide.

The kinds of microorganisms of the present invention are not limited to those referred herein and may be any kind of microorganisms that can be used in peptide production. In this specification, the microorganisms may include eucaryotic microorganisms, prokaryotic microorganisms, and viruses. From the viewpoint of application to industrial production, easiest-to-treat bacteria are preferable, enterobacteria are further preferable, and particularly, bacteria that belong to the genus *Escherichia,* of which typical microorganism is *Escherichia coli,* are furthermore preferably exemplified.

Now, giving an example of a mode, wherein decomposition activity of alanyl-glutamine and aspartylphenylalanine is decreased or deactivated, the method for preparing the strains of disrupted genes is more specifically explained. The decomposition activity of alanylglutamine and aspartylphenylalanine may be decreased or deactivated by inducing mutation in the gene encoding each of peptidases or by deactivating the expression of the gene, so that the activity of peptidases acting on alanylglutamine and aspartylphenylalanine as well as their derivatives may be decreased or deactivated in cells. To deactivate peptidase genes, commonly-used techniques can be used including UV irradiation, mutation induction process with nitrosoguanidine (N-methyl-N'-nitro-N-nitrosoguanidine), site-specific mutation, homologous replacement, and/or gene disruption using insertion-deletion mutation also called "Red-driven integration"(Datsenko K.A. and Wanner B.L., Proc. Natl. Acad. Sci. USA, 2000, Vo1.97, No.12, p6640-45).

The Peptidases that decompose alanylglutamine and aspartylphenylalanine as well as their derivatives include; aminoacylhistidine dipeptidase encoded by a *pepD* gene; α-aspartyldipeptidase encoded by a *pepE* gene; leucylaminopeptidase encoded by a *pepA*; and isoaspartyldipeptidase encoded by an *iadA* gene. It has been known that the *pepD, pepE, pepA*, and *iadA* genes are contained in the microorganisms, for example, *Escherichia coli* (*pepD* Gene;J. Bacteriol. 172 (8), 4641-4651 (1990), *pepE* Gene; JP-A No. 189863/2003, *pepA* Gene;J Mol Biol. 2000 Sep 15;302(2):411-26, *iadA* Gene;J Biol Chem. 1995 Feb 24;270(8):4076-87). The deficient strain lacking each peptidase gene may be obtained by, for example, homogeneously recombining the DNA fragment containing a region of each gene with its corresponding gene on the chromosome of the microorganism to integrate them into the chromosome DNA.

Specifically, peptidase genes on the chromosome may be deleted by, for example, transforming microorganisms such as *Escherichia coli* with the DNA containing the deficient peptidase gene, wherein the region of the peptidase gene has been deleted to alter so that normally working peptidase may be prevented from being produced, which induces recombination of the deficient peptidase gene with the peptidase gene on the chromosome. The method for deleting genes using the aforementioned homologous replacement technique has been well-established. Alternatively, the gene may be deleted by any of the methods using a liner DNA and using plasmid containing temperature-sensitive replication origin, and/or by the insertion-deletion mutation method also called "Red-driven integration". The method using plasmid containing the temperature-sensitive replication origin and the insertion-deletion mutation method also called "Red-driven integration" are explained below.

The nucleotide sequences of *pepD, pepE, pepA*, and *iadA* genes encoding peptidases contained in *Escherichia coli* have been registered in DDBJ/EMBL/GenBank International Nucleotide Sequence Database with accession numbers AE000132, AE000475, AE000496, and AE000503 assigned, respectively. It is possible to obtain fragments of each gene by synthesizing primers based on these nucleotide sequences and replicating them using chromosome DNAs of *Escherichia bacteria,* for example, *Escherichia coli* W3110 strain as templates by a Polymerase Chain reaction (PCR; White,T.J. et al., Trends Genet., 5,185 (1989)).

Then, the DNA containing the gene, wherein some part of the nucleotide sequence thereof has been lost (deficient peptidase gene) to alter so that normally-working peptidase may be inhibited from being produced using the obtained peptidase gene as a material. To replace this deficient peptidase gene for the peptidase gene expressed on the host chromosome, the procedure described below is followed. That is, the transformed strain, wherein the recombinant DNA has been integrated into the chromosome DNA by following the steps for; incorporating the temperature-sensitive replication origin, the mutant peptidase gene, and the marker gene having resistance to antibiotics such as ampicillin, kanamycin, tetracycline and chloramphenicol to prepare the recombinant DNA; transforming the obtained recombinant DNA; cultivating the transformed strain at such a temperature that the temperature-sensitive replication origin is not activated; and cultivating the strain in the medium.

The strain having the recombinant DNA integrated in the chromosome in this way has been replaced for a native peptidase gene sequence on the chromosome and two fusion genes coupling the chromosome peptidase gene and the deficient peptidase gene are contained in the chromosome with the remaining region of the recombinant DNA (a vector region, the temperature-sensitive replication origin, and the drug-resistance marker) laid between them. Thus, since the normal peptidase gene has dominance in this state, the peptidase expresses in the transformed strain.

Second, to keep only the deficient peptidase gene on the chromosome DNA, two peptidase genes are recombined to remove one copy of peptidase gene with the vector region (including the temperature-sensitive replication origin and the drug-resistance marker) from the chromosome DNA. Note that in some cases, the normal peptidase genes may remain on the chromosome DNA and the deficient peptidase genes may be separated, while in other cases, the deficient peptidase genes may be left on the chromosome gene and the normal peptidase genes may be separated. In any case, the cultivation of the tranformants at such a temperature that the temperature-sensitive replication origin is activated allows the separated DNA to remain in the cell in the state of plasmid.

Third, the cultivation of the transformants at such a temperature that the temperature-sensitive replication origin is not activated, decreases or inhibits the peptidase activity because plasmid containing the deficient peptidase genes is removed, if the deficient peptidase genes remain on the chromosome DNA, while peptidase activity is activated if the normal peptidase genes remain on the chromosome DNA. The target strain may be selected by amplifying the fragment containing the peptidase genes from the chromosome DNA of a candidate strain through Polymerase Chain Reaction (PCR) and by verifying that the peptidase genes have been knockout by restriction endonuclease analysis.

Alternatively, the target peptidase genes may be deleted by the Red-driven integration method (Proc. Natl. Acad. Sci. USA, 2000, vol. 97, No. 12, p6640-6645). According to the method described in the publication, primers are designed having complementary sequence to the region in the vicinity of each of genes; the peptidase gene; and the gene providing antibiotic-resistance to the template plasmid. For example, PCR is performed using the plasmid pACYC184 (NBL Gene Sciences Ltd.(England), GenBank/EMBL Accession No. X06403) as the template and the primers complementary to the region in the vicinity of each of the genes; the peptidase gene; and the gene for providing chloramphenicol-resistance to the template plasmid; to prepare the gene inserted the gene for providing chloramphenicol-resistance into the peptide gene to be deleted. Then, the resultant PCR product is purified in agarose gel and using this purified product, *Escherichia coli* containing plasmid pKD46 having an ability to replicate the temperature-sensitivity property is transformed by electroporation. Plasmid pKD46 (Proc. Natl. Acad. Sci. USA, 2000, vol. 97, No. 12, p6640-6645) contains the DNA fragment GenBank/EMBL accession Nos. J02459, 31088^{th} to 33241^{th}) of 2154 nucleotides of λ phage containing the genes (λ, β, and exo genes) in a λRed homologous recombinant system controlled by an arabinose-derivative ParaB promoter. Plasmid pKD46 is necessary to integrate the PCR product into the chromosome of *Escherichia coli*.

Competent cells for electroporation may be prepared in the method mentioned below. Specifically, *Escherichia coli*, that has been cultivated overnight in the LB medium containing 100 mg/L of ampicillin at 30°C, is 100-fold diluted with 5 mL of SOB medium (Molecular Cloning: Laboratory Manual 2^{nd} version, Sambrook, J.et al., Cold Spring Harbor Laboratory Press (1989)) containing ampicillin and L-arabinose (1 mM). The resultant diluted solution may be used in electroporation by following the steps for; growing the cells at 30°C until OD₆₀₀ reaches an approximate point of 0.6 while aerating; concentrating the solution to a 100-fold concentration; and rinsing the cells with deionized water cooled on ice three times. The mutant microorganisms with the peptidase genes deleted may be obtained through an insertion-deletion mutation process, wherein electroporation is performed using 70 µL of competent cells and approximately 100 ng of PCR products, the cells are plate-cultured in the L-agar medium containing chloramphenicol at 37°C and a chloramphenicol-resistant recombinant is selected. Moreover, pKD46 plasmid may be removed by cultivating the obtained recombinant in the L-agar medium containing chloramphenicol over two generations at 42 °C and selecting a colony with the ampicillin-resistance lost.

As to the mutant with the peptidase gene deleted, which has been obtained in the manner mentioned above and may be identified by the chloramphenicol-resistant gene, the target strain may be confirmed by amplifying the fragment containing the peptidase genes from the chromosome DNA of a candidate strain through PCR and by verifying that the peptidase genes have been deleted by the method such as restriction endonuclease analysis. Thus, the strains with the genes disrupted may be prepared.

According to another preferable embodiment of the present invention, the microorganism, which has been transformed by further introducing the recombinant DNA containing polynucleotide encoding proteins having peptide-synthesizing activity into the gene disrupted strain, which have been prepared in the aforementioned manner, may be obtained. According to further another preferable embodiment of the present invention, the proteins derived from a bacterium belonging to the genus of *Empedobacter brevis* or *Sphingobacterium* is used as the proteins having peptide-synthesizing activity. More specifically, it is preferable that one or more of proteins selected from the group consisting of proteins (A) to (D) mentioned below are used. The proteins of groups (A) to (D) mentioned below have superior peptide-synthesizing activity. Moreover, the proteins of (A) to (D) groups are suitable for forming peptides such as aspartylphenylalanine and alanylglutamine.
(A) a protein comprising an amino acid sequence consisting of amino acid residues numbers 23-616 of an amino acid sequence in SEQ ID NO: 14
(B) a protein comprising an amino acid sequence including substitution, deletion, insertion, addition, and/or inversion of one or several amino acid residues in the amino acid sequence consisting of amino acid residues numbers 23-616 of an amino acid sequence in SEQ ID NO: 14, and having the peptide-synthesizing activity
(C) a protein comprising an amino acid sequence consisting of amino acid residues numbers 21-619 of an amino acid sequence in SEQ ID NO: 20
(D) a protein comprising an amino acid sequence including substitution, deletion, insertion, addition, and/or inversion of one or several amino acid residues in the amino acid sequence consisting of amino acid residues numbers 21-619 of an amino acid sequence in SEQ ID NO: 20, and having the peptide-synthesizing activity.

An entire range of amino acid sequences categorized in SEQ ID NO: 14 contains a leader peptide and a mature protein domain, the leader peptide consisting of amino-acid residues with NOs. 1 to 22 and the mature protein domain consisting of those with NOs. 23 to 616.

An entire range of amino acid sequences categorized in SEQ ID NO: 20 contains the leader peptide and the mature protein domain, said leader peptide consisting of amino-acid residues with NOs. 1 to 20 and said mature protein domain consisting of those with NOs. 21 to 619.

Herein, the term "several" refers to the number of amino acids within a range, which have less effect on the three-dimensional structure of the protein composed of amino-acid residues and the activity of the protein. It may be depending on the location and the types of amino-acid residues in the three-dimensional structure of the protein, and the range is specifically 2 to 50, preferably 2 to 30, and several preferably 2 to 10. Note that it is desirable that among those of protein groups (B) and (D), the protein comprising the amino-acid sequence containing substitution, deletion, insertion, addition, and/or inversion of one or several amino acid residues, retain approximately 50% or more, preferably 80% or more, and more preferably 90% or more of enzyme activity of the protein comprising no mutation under the conditions, 50°C and pH8. Giving the amino acid sequence in SEQ ID. NO: 6, (B) as an example, the protein comprising amino acid sequence containing substitution, deletion, insertion, addition, and/or inversion of one or several amino acid residues, desirably retain approximately 50% or more, preferably 80% or more, and more preferably 90% or more of enzyme activity of the protein comprising the amino acid sequence in SEQ ID. NO: 6 under the conditions, 50°C and pH8.

The mutation in amino acids as shown in the aforementioned (B) may be induced by modifying the nucleotide sequence so that the amino acids at the corresponding sites of the enzyme gene may be substituted, deleted, inserted, and/or added using, for example, the site-specific mutation method. Alternatively, the polynucleotide having modified nucleotide sequence may be obtained by the conventionally known the mutation process. The mutation process may include a technique involving a step for *in vitro* inducing the mutation in the DNA encoding the amino acids of the (A) or (C) under the treatment of hydroxylamine and a technique involving a step for introducing the mutation in a bacterium belonging to the genus *Escherichia* containing the DNA encoding the amino acids of the (A) or (C) group by UV irradiation, or under the treatment with methyl-N'-nitro-N-nitrosoguanidine(NTG)or any of mutating agents generally used for mutation engineering, such as nitrous acid.

The mutations such as the aforementioned substitution, deletion, insertion, addition and/or inversion in nucleotides include inherent mutation induced by a difference among the species of microorganisms or the strains and mutation induced naturally. By causing the DNA having the aforementioned mutation in an appropriate cell to express to examine the enzyme activity of an expressing product, the DNA encoding the substantially same protein as those categorized in SEQ ID NO: 14 or 20 in the Sequence Listing may be obtained.

The amino acid sequence contained in the aforementioned proteins of the (A) is encoded based on, for example, the nucleotide sequences SEQ ID NO: 13. The DNA consisting of the nucleotide sequences with BASE NOs. 61~1908 in SEQ ID NO: 13 has been isolated from *Empedobacter brevis* FERM BP-8113 strain deposited at International Patent Organism Depository, the National Industrial Research Institute (1-1 Chuoh 6, Higashi 1-Chome, Tsukuba-shi, Ibaraki-ken, Japan) on July 8, 2002.

The DNA consisting of the nucleotide sequences with BASE NOs. 61~1908 in SEQ ID NO: 13 corresponds to a code sequence (CDS) region. The nucleotide sequence with BASE NOs. 61~1908 contain the signal sequence and mature protein domains. The signal sequence domain is a region ranging from BASE NOs. 61 to 126 and the mature protein domain is a region ranging from BASE NOs. 127 to 1908. Namely, the present invention provides both of peptide enzyme protein genes containing the signal sequence and peptide enzyme protein genes as mature proteins. The signal sequence included in the nucleotide sequence in SEQ ID NO: 13 is one kind of leader sequence, wherein the main function of the leader peptide encoded by the leader sequence is possibly to cause peptide to be secreted out of a cell membrane from inside of the cell membrane. The protein encoded based on BASE NOs. 127 to 1908, which is the region excluding the leader peptide, corresponds to the mature protein which possibly has high peptide-synthesizing activity.

The amino acid sequence contained in the aforementioned protein of (C) is encoded based on, for example, the nucleotide sequences in SEQ ID NO: 19. The DNA consisting of the nucleotide sequence with BASE NOs. 61 to 1917 categorized in SEQ ID NO: 19 has been isolated from *Sphingobacterium SP* FERM BP-8124 strain deposited at International Patent Organism Depository, the National Industrial Research Institute (1-1 Chuoh 6, Higashi 1-Chome, Tsukuba-shi, Ibaraki-ken, Japan) on July 22, 2002. The DNA consisting of the nucleotide sequences with BASE NOs. 61 to 1917 in SEQ ID NO: 11 corresponds to the code sequence (CDS) region.

The nucleotide sequence with BASE NOs. 61 to 1917 contains the signal sequence and mature protein domains. The signal sequence domain is a region ranging from BASE NOs. 61 to 120 and the mature protein domain is a region ranging from BASE NOs. 121 to 1917. Namely, the present invention provides both of the peptide enzyme protein gene containing the signal sequence and the peptide enzyme protein gene as a mature protein. The signal sequence included in the nucleotide sequence in SEQ ID NO: 19 is one kind of leader sequence, wherein the main function of the leader peptide encoded in the leader sequence domain is possibly to cause the peptide to be secreted out of the cell membrane from inside of the cell membrane. The protein encoded based on BASE NOs. 121 to 1917, which is the region excluding the leader peptide, corresponds to the mature protein which possibly has high peptide-synthesizing activity.

The DNA of SEQ ID NO: 13 and the DNA of SEQ ID NO: 19 may be obtained from the chromosome DNAs of *Empedobacter brevis* and *Sphingobacterium SP,* or DNA libraries, respectively through PCR (polymerase chain reaction, refer to White,T.J. et al;Trends Genet., 5, 185 (1989)) or hybridization. The primers to be used in PCR may be designed by using internal amino acid sequences determined based on the purified protein having peptide-synthesizing activity. In addition, a probe for hybridization other than the primer may be designed, and the DNA of SEQ ID NOs:13 and 19 may be isolated using each probe. Using the primers having the sequences corresponding to 5'and 3' non-translation domains, respectively, as those for PCR, the whole length of encoding domain of the protein may be amplified. Giving as an example such a case that the domain containing both the leader sequence and mature protein encoding domains is amplified, specifically, the primers for PCR may include the primer having nucleotide sequences in the upstream of the nucleotide sequence with BASE NOs. 61 in SEQ ID NO: 13 for the primer on the 5' side and the primer having nucleotide sequences complementary to those in the downstream of the nucleotide sequence with BASE NOs. 1908 as the primer for the 3' side.

The primer may be synthesized by, for example, the phosphoramidite method (refer to Tetrahedron Letters (1981),22,1859)using a DNA synthesizer model 380B supplied from Applied Biosystems in the usual manner. The PCR may be performed by the method specified by the suppliers, for example, the manufacturers of Gene Amp PCR System 9600 (PERKIN ELMER) and TaKaRa LA PCR in vitro Cloning Kit (TaKaRa).

In addition to the DNA having nucleotide sequence in SEQ ID NO: 13, the DNA substantially the same as that composed of CDS in SEQ ID NO: 13 may be used, regardless of the presence of the leader sequence. Namely, substantially the same DNA in SEQ ID NO: 13 may be also obtained from the DNA encoding the enzyme having the mutation or the cell retaining this DNA by hybridizing with the probe, which is prepared the DNA consisting of the nucleotide sequence complementary to CDS in SEQ ID NO: 13 or the same nucleotide sequence under a stringent condition, and by isolating the DNA encoding the proteins having the peptide-synthesizing activity.

In addition to the DNA having nucleotide sequence in SEQ ID NO: 19, the DNA substantially the same as that composed of CDS in SEQ ID NO: 19 may be used, regardless of the presence of the leader sequence. Namely, substantially the same DNA in SEQ ID NO: 19 may be also obtained from the DNA encoding the enzyme having the mutation or the cell retaining this DNA by hybridizing under a stringent condition with the probe, which is prepared based on the DNA consisting of the nucleotide sequence complementary to CDS in SEQ ID NO: 19 or the same nucleotide sequence, and by isolating the DNA encoding the proteins having the peptide-synthesizing activity.

The probe may be prepared based on, for example, the nucleotide sequence in SEQ ID NO: 19 in the usual manner. Alternatively, for a target DNA to be isolated, the prove is used to hybridize with and probe the DNA in the usual way. For example, the DNA probe may be prepared by amplifying the nucleotide sequence cloned into the plasmid or phage vector, followed by separation and extraction of the nucleotide sequence to be used as the prove with restriction enzyme. The site, at which the nucleotide sequence is separated, may be regulated depending on the target DNA.

The term "under a stringent condition" as used herein refers to a condition under which a so-called specific hybrid is formed but no non-specific hybrid is formed. It is difficult to precisely express this condition in numerical values. For example, mention may be made of a condition under which DNAs having a high homology, for example, 50% or more, preferably 80% or more, more preferably 90% or more, hybridize with each other and DNAs having a lower homology than these do not hybridize with each other, or ordinary conditions for rinse in southern hybridization under which hybridization is performed at 60°C in a salt concentration corresponding to 1×SSC and 0.1% SDS, preferably 60°C, 0.1×SSC, and 0.1% SDS. The activity of peptide-synthesizing enzyme is as already explained above. In some of genes hybridized under any of these conditions, a stop codon may exist in the sequence or their activity may be lost due to the mutation at an active center. These genes may be easily removed by coupling them to the vectors commercially available, which causes them in an appropriate host to express, followed by determining the enzyme activity of an expressing product in the manner mentioned later.

However, in the case of the nucleotide sequence that hybridizes with a complementary nucleotide sequence under a stringent condition, it is desirable that the protein encoded thereby retain an enzyme activity of 10% or more, more preferably 50% or more of the enzyme activity of the protein having the original amino acid sequence be retained under conditions of 50°C and pH 8. For example, the nucleotide sequence, which is hybridized under the stringent condition with a DNA consisting of nucleotide sequence complementary to the nucleotide sequence with BASE NOs. 127 to 1908 in SEQ ID NO: 9 are provided as an example. It is desirable that the DNA retains the enzyme activity in approximately 50% or more, preferably 80% or more, and more preferably 90% or more of the protein having the amino acid sequences with amino-acid residue NOs. 23 to 616 in SEQ ID NO: 6 under the conditions, 50°C and pH8.

The method for producing the microorganisms transformed so that the proteins aforementioned in (A) or (C) may express. The transformant, which may express the protein aforementioned in (A) or (C) having the peptide-synthesizing activity, may be formed by, for example, introducing the DNA having nucleotide sequence in SEQ ID NO: 13 or 19 into an appropriate host to allow them to express.

The host, which may be used as the host where the protein identified by the DNA having nucleotide sequence in SEQ ID NO: 13 or 19 express, may include any of bacteria of the genera of *Escherichia* such as *Escherichia coli,* of *Empedobacter,* of *Sphingobacterium,* and of *Flavobacterium;* a wide variety of procaryotic cells including *Bacillus subtilis*; and a wide variety of eucaryotic cells including *Saccharomyces cerevisiae, Pichia stipitis,* and *Aspergillus oryzae*.

The recombinant DNA, which is used to introduce the DNA having nucleotide sequence in SEQ ID NO: 13 or 19 into the host, may be prepared by introducing the DNA into the appropriate vector depending on the type of the host in such a mode that the protein to be encoded by the DNA may express. For a promoter, which mediates protein expression, the specific promoter for the peptide-synthesizing enzyme gene in the microorganism such as *Empedobacter brevis,* may be used, provided that it works normally in the host cell. Alternatively, if necessary, any other promoter, which works normally in the host cell, may be ligated to the DNA in SEQ ID NO: 13 or 19 to allow the proteins to express under the control of the promoter.

The transformation method for introducing the recombinant DNA into the host cell includes the D.M.Morrison's method (Methods in Enzymology 68, 326 (1979)) and the method involving treating recipient bacterium cells with calcium chloride to enhance DNA permeability (Mandel,M. and Higa,A.,J.Mol.Biol.,53,159 (1970)).

When a protein is mass-produced by using the recombinant DNA technology, a preferable embodiment includes a mode in which the protein molecules associate to form inclusion body of protein in the transformant that produces the protein. This expression production method has an advantage, for example, in that the target protein is protected from digestion by proteases that exist in the microbial cells and that the target protein can be easily purified by disrupting the microbial cells and subsequent centrifugation operation.

The inclusion body of protein thus obtained is solubilized with a protein modifier and converted into a physiologically active protein that is properly folded after passing through an activating reconstitution operation by removing the modifier. There are many examples including, for example, activating reconstitution of human interleukin-2 (JP 61-257931A).

To obtain an activated type protein from the inclusion body of protein, a series of operations such as solubilization and activating reconstitution are necessary, so that the operation becomes more complicated than the case in where the activated type protein is directly produced. However, in the case where a protein that influences on the growth of microbial cells is mass-produced in the cells, the influence may be suppressed by allowing the protein to be accumulated in the cells in the form of an inactive inclusion body of protein.

The method in which a target protein is mass-produced as an inclusion body includes a method in which the target protein is allowed to be expressed alone under control of a potent promoter, and a method in which the target protein is allowed to be expressed as a fused protein with a protein whose mass expression has been known.

Giving an example, a method, which involves a step for preparing transformed *Escherichia coli* followed by preparing the peptide-synthesizing enzyme using the same bacterium, is more specifically mentioned below. Note that to allow the microorganisms such as *Escherichia coli* to form the peptide-synthesizing enzyme, the DNA, which encodes a precursor protein containing a leader sequence, may be combined as a protein coding sequence or only the DNA in the mature protein domain with no leader sequence contained may be combined. Any of these methods may be selected at one's discretion taking into account the conditions for production and usage, and the morphology of the enzyme to be produced and other factors.

For the promoter, which mediates the expression of the DNA encoding the proteins having the peptide-synthesizing activity, the promoter may be usually used in producing heterogeneous proteins in the microorganism of *Escherichia coli.* The promoter includes powerful promoters such as T7 promoter, lac promoter, trp promoter, trc promoter, tac promoter, PR promoter of λphage and PL promoter. For the vector, any of pUC19, pUC18, pBR322, pHSG299, pHSG298, pHSG399, pHSG398, RSF1010, pMW119, pMW118, pMW219, and pMW218 may be used. Moreover, the vector of the phage DNA may be used. Further, an expression vector containing the promoter and capable of inducing the expression of inserted DNA sequences may be used.

To produce the peptide-synthesizing enzyme as a fused protein inclusion body, the gene encoding any other protein, preferably hydrophilic peptides is ligated to the upstream or downstream of the gene for the peptide-synthesizing enzyme. For the gene encoding aforementioned other proteins, any of genes, which have the ability to increase the accumulation level of fused proteins and enhance the solubility of denaturalized and reproduced proteins, may be used. The candidate for such genes include, for example, T7gene 10, β-galactosidase gene, dehydrofolate reductase gene, interferony gene, interleukin2 gene, and prochymosin gene.

To ligate any of these genes to the gene encoding the peptide-synthesizing enzyme, the reading frames of codons need to be aligned. To achieve it, these genes may be ligated at an appropriate site of restriction enzyme, or a synthesize DNA consisting of appropriate nucleotide sequences may be used.

In some cases, a terminator, namely a transcription termination sequence, is preferably linked to the downstream of the fused protein gene to increase the yield of proteins. Such terminators include, for example, T7 terminator, fd phage terminator, T4 terminator, tetracycline-resistant gene terminator and Escherichia coli trpA gene terminator.

It is preferable that the vector, which may be used in introducing into *Escherichia coli* the protein having the peptide-synthesizing activity or the gene encoding the fused protein consisting of the proteins having the peptide-synthesizing activity fused with other protein, is the so-called multi-copy type of vector and includes ColE1-derived plasmids having the replication origin, for example, pUC and pBR322 line plasmids or their derivatives. Herein, the term "derivatives" mean the plasmids modified by substitution, deletion, insertion, addition and/or invertion of the nucleotides. Note that herein, "modified" includes modification by the mutation techniques with a mutagenic agent or UV irradiation, or through the natural mutation process.

Furthermore, to select out the transformant, the vector preferably have the marker for, for example, the ampicillin-resistant gene. Expression vectors having the powerful promoters as these plasmids, are commercially available (pUC line (supplied from TaKaRa), pPROK line (supplied from Clontech), pKK233-2 (supplied from Clontech) and others).

The recombinant DNA may be obtained by ligating the DNA fraction formed through the linkage of the promoter, the gene encoding the protein having the peptide-synthesizing activity or the fused protein consisting of the protein having the peptide-synthesizing activity and other protein, and in some cases, a terminator to a vector DNA in that order.

Using the resulting recombinant DNA, transformation of, e.g., *Escherichia coli* may be performed. Cultivation of this *Escherichia coli* may result in expression and production of the peptide-synthesizing enzyme or the fusion protein of the peptide-synthesizing enzyme and the other protein. The host to be transformed may be strains that are usually employed for the expression of foreign genes. Preferable examples thereof may include *Escherichia coli* JM109 strain, a subspecies of *Escherichia coli* K12. Methods for performing transformation and methods of selecting the transformant are described in Molecular Cloning, 2nd edition, Cold Spring Harbor Press (1989) and the like.

In the case of expressing the enzyme as a part of the fusion protein, the fusion protein may be composed so as to be able to cleave the peptide-synthesizing enzyme therefrom using a restriction enzyme which recognizes a sequence of blood coagulation factor Xa, kallikrein or the like which is not present in the peptide-synthesizing enzyme.

The production media to be used may include the media usually used for culturing *Escherichia coli,* such as M9-casamino acid medium and LB medium. Culture conditions and production induction conditions may be appropriately selected depending on types of the vector marker, the promoter, the host bacterium and the like.

The peptide-synthesizing enzyme or the fusion protein of the peptide-synthesizing enzyme and the other protein may be recovered by the following method: when the peptide-synthesizing enzyme or the fusion protein of the peptide-synthesizing enzyme is solubilized in the microbial cells, the microbial cells may be recovered and then disrupted or lysed, to obtain a crude enzyme solution. If necessary, the peptide-synthesizing enzyme or the fusion protein may further be subjected to purification in accordance with ordinary methods such as precipitation, filtration and column chromatography. The purification may also be performed in accordance with methods utilizing an antibody against the peptide-synthesizing enzyme or the fusion protein.

In the case where the protein inclusion body is formed, this may be solubilized with a denaturing agent. The inclusion body may be solubilized together with the microbial cells. However, considering the following purification process, it is preferable to remove the inclusion body before solubilization. Collection of the inclusion body from the microbial cells may be performed in accordance with conventionally and publicly known methods. For example, the microbial cells are disrupted, and the inclusion body is recovered by centrifugation and the like. The denaturing agent which solubilizes the protein inclusion body may include guanidine-hydrochloric acid (e.g., 6 M, pH 5 to 8), urea (e.g., 8 M), and the like.

As a result of removal of the denaturing agent by dialysis and the like, the protein may be regenerated as having the activity. Dialysis solutions used for the dialysis may include tris hydrochloric acid buffer, phosphate buffer and the like. The concentration thereof may be 20 mM to 0.5 M, and pH thereof may be 5 to 8.

It is preferred to keep that the protein concentration at a regeneration step is kept at about 500 µg/ml or less. In order to inhibit self-crosslinking of the regenerated peptide-synthesizing enzyme, it is preferred that dialysis temperature is kept at 5°C or below. Methods for removing the denaturing agent other than the dialysis method may include a dilution method and an ultrafiltration method. The regeneration of the activity is anticipated by using any of these methods.

By transforming the microorganism, of which *pepD, pepA, iadA*, or *pepE* gene has been disrupted in the aforementioned manner, the microorganism with the increased expression of amino-acid ester transpeptidase and the reduced or lost activity of decomposing alanylglutamine and aspartylphenylalanine may be produced.

### 2. Method for producing peptides of the present invention

The method for producing the peptides of the present invention involves forming the peptides using the aforementioned microorganisms of the present invention. Namely, the method for producing the peptides of the present invention involves the step for cultivating the microorganism, of which at least one gene on the chromosome selected from the group consisting of genes encoding aminoacylhistidine peptidase, luecylaminopeptidase, and isoaspartyldipeptidase has been disrupted and which has been transformed with a recombinant DNA containing the polynucleotide encoding the proteins having the peptide-synthesizing activity, in the medium, followed by mixing at least one of the cultivated microorganisms and the disrupted cells thereof with carboxy and amine components to synthesize peptides. According to further another preferable mode, for the aforementioned microorganism, the microorganism, of which gene encoding α-aspartyldipeptidase on the chromosome has been further disrupted.

For the microorganism used in the present invention to act on the carboxy and amine components, the aforementioned microorganisms or the disrupted cells of the microorganisms may be mixed with these components. More specifically, the microorganism may be added into the solution containing carboxy and amine components to induce the reaction. Alternatively, such a method may be used that the microorganism having the ability to form the target peptides is cultivated, the enzyme is formed and accumulated in the microorganisms or the culture fluid of the microorganism, and the carboxy and amine components are added into them. The formed peptides may be recovered in the usual manner and purified if necessary.

Herein, the term "disrupted cells of microorganisms" refers to a mixture containing the contents of a cell obtained by disrupting the cell membrane of the microorganism. It includes not only the contents of the cell obtained by physically disrupting the cell membrane but also that obtained by chemically dissolving the cell membrane.

The microorganism of the present invention, in which the specific gene has been disrupted, is used. In producing the peptides, not only the microorganism cells, but also the treated microorganism cell products such as acetone-treated microorganism cells or freeze-dried microorganism cells may be used as the microorganism. Alternatively, the microorganism cells or the treated microorganism cell products immobilized by any of the covalent binding method, the absorption method, the entrapment method, and so forth may be used. Note that in many cases, there is an enzyme dissolving the formed peptides rather than involving in peptide formation. In such a case, any of metal-protease inhibitors such as ethylenediamine tetraacetic acid (EDTA) may be preferably added. The quantity of added inhibitor is 0.1 mM to 300 mM, and preferably ranges from 1 mM to 100 mM.

As the carboxy component, any of substrates capable of condensing with the amine component, another substrate, to form peptides may be used. The carboxy component includes, for example, L-amino-acid ester, D-amino-acid ester, L-amino-acid amide, D-amino-acid amide, and organic-acid ester with no amino group. The examples of amino-acid esters include not only amino acid ester corresponding to natural amino acid but also that corresponding to non-natural amino acid or to its derivatives. Further, the examples of amino-acid esters include not only α-amino-acid ester but also β-, γ-, and ω- amino-acid esters, which have different amino group binding sites. The typical examples of amino-acid esters may include, for example, methyl ester, ethyl ester, n-propyl ester, isopropyl ester, n-butyl ester, isobutyl ester, and tert-butyl ester of amino acids.

As the amine component, any of substrates capable of condensing with the carboxy component, another substrate, to form the peptides may be used. The amine component includes, for example, L-amino acid, C-protected L-amino acid, D-amino acid, C-protected D-amino acid, and amine. Further, the examples of amines include not only natural amine but also non-natural amine or its derivatives. Furthermore, the examples of amino acids include natural amino acid but also non-natural amino acid or its derivatives. Furthermore, the examples of amino acids include not only α-amino acid but also β-, γ-, and ω-amino acid, which have different amino group binding sites.

The concentrations of carboxy and amine components, starting materials, are 1 mM to 10 M, respectively, and preferably 0.05 M to 2M, respectively, In some cases, however, more than equal amount of amine component may be preferably added compared with the carboxy component. If the high-concentration of substrates inhibits the reaction, the substrates may be added bit by bit at concentrations, which do not inhibit the reaction.

The peptides may be synthesized at the reaction temperature ranging from 0 to 60°C, and preferably 5 to 40°C. The peptides may be synthesized at the reaction pH value ranging from pH 6.5 to 10.5 and preferably, pH7.0 to 10.0.

### EXAMPLE

The examples for carrying out the present invention are explained below. However, the present invention is not limited to the following examples for carrying out the present invention.

### Example 1. Determination of aspartylphenylalanine decomposition activity in each of peptidase gene-deficient strains

In the process mentioned below, the *Escherichia coli* ATCC8739 strain was used as a parent strain. Aminoacylhistidine dipeptidase gene (*pepD* gene), isoaspartyldipeptidase gene (*iadA* gene), α-aspartyldipeptidase gene (*pepE* gene), leucylaminopeptidase gene (*pepA* gene) of the parent strain were disrupted in that order to construct the *pepD*-deficient strain, the *pepD-iadA* double deficient strain, the *pepD-iadA-pepE* triple deficient strain, and the *pepD-iadA-pepE-pepA* quadruple deficient strain, and aspartylphenylalanine decomposition activity of them were determined.

### (1) Disruption of aminoacylhistidine dipeptidase gene (pepD gene)

Based on sequence information on aminoacylhistidine dipeptidase gene (pepD gene) (DDBJ/EMBL/GeneBank Accession No. AE000132) in Gene Data Bank, the primers of 5'-GATCTGGCGCACTAAAAACC (SEQ ID NO: 1) and 5'-GGGATGGCTTTTATCGAAGG (SEQ ID NO: 2) were synthesized. The primers were used to amplify approximately 1.6 Kbp of structural gene, which covered SD-ATG and a translation termination codon by the PCR method (94°C, 1 min, 54°C, 2 min, 72°C, 3 min, 30 cycles) using a genome DNA of the *Escherichia coli* ATCC8739 strain as a template. Using SureClone Ligation Kit (supplied from Amersham Pharmacia Biotech), the amplified structural gene was ligated to the *Sma* I site of a pUC18 (supplied from TaKaRa) vector to form a pUC18-pepD gene. The *Escherichia coli* JM109 competent cells (supplied from TaKaRa) were transformed with the plasmid and grown on the LB agar plate(Tryptone 1%, Yeast extract 0.5%, NaCl 0.5%, agar 1.5%, pH7.0) containing 50µg/ml of ampicillin. The tranformant was cultivated in the LB liquid medium (Tryptone 1%, Yeast extract 0.5%, NaCl 0.5%, pH7.0) containing 50µg/ml of ampicillin at 37°C for 16 hours to prepare the plasmid from recovered transformant. Then, pUC18-pepD was cleaved at *Nru* I and *Hpa* I sites (the restriction enzyme sites in 1.6 Kbp of the inserted fragment containing the *pepD* gene) and self-ligation reaction was induced. Using the ligation reaction mixture, the competent cells of the *Escherichia coli* JM109 strain were transformed and the plasmid was prepared from the transformant growing on the LB agar plate containing 50µg/ml of ampicillin. From resultant products, the plasmid DNA, pUCΔ*pepD* having 1.4 Kbp of the inserted DNA fragment, was selected. It is expected that the *pepD* gene ligated to the plasmid might lose the *Nru* I-*Hpa* I region and the enzyme to be encoded has been inactivated.

Then, pUCΔ*pepD* was cleaved at the *Eco*R I and *Sal* I sites to prepare 1.4 Kbp of DNA fragment containing the deficient *pepD* gene. The DNA fragment was ligated to the *Eco*R I/*Sal* I sites of pMAN997 having the temperature-sensitive replication origin (tsori), the vector for homologous replacement, to form the plasmid for deficient pMANΔ*pepD* gene. The *Escherichia coli* JM 109 strain, a subspecies of the *Escherichia coli* K12 strain transformed with pMAN*ΔpepD*, was cultivated in the LB liquid medium containing 50µg/ml of ampicillin at 30 °C for 16 hours to prepare pMANΔ*pepD* from the recovered transformant.

Then, using the plasmid pMANΔ*pepD* for the deficient *pepD* gene, the *Escherichia coli* ATCC8739 strain was transformed by electroporation and grown in the LB agar plate containing 50µg/ml of ampicillin at 30°C. (Note that among the aforementioned strains, those with ATCC numbers have been deposited in American Type Culture Collection (P.O.Box 1549 Manassas, VA 20110, the United States of America) and may be furnished by referring to the respective numbers.) A plurality of obtained transformants were cultivated in 4 ml of LB medium containing 50µg/ml of ampicillin (in a ϕ1.4 cm×18 cm test tube) at 30°C for 16 hours. The culture solution was diluted with physiological saline, applied into the LB agar plate containing 50µg/ml of ampicillin, and cultivated at 42°C for 10 hours to obtain a single colony. Further, the obtained single colony was cultivated again in the same manner as the aforementioned one to isolate the single colony, and a clone with the entire plasmid integrated in the chromosome by homologous replacement was selected. Furthermore, the plasmid was extracted from the homologous recombinant strain cultivated in the LB medium containing 50µg/ml of ampicillin and it was verified that the strain had no plasmid in the cytoplasmic solution.

Then, 10 clones were selected from those with the entire plasmids incorporated in the chromosomes by homologous replacement in the aforementioned manner and cultivated in the M9 minimum liquid medium (4 ml,Na₂PO₄ 6.8 g, KH₂PO₄ 3 g, NaCl 0.5 g, NH₄Cl 1 g, MgSO₄·7H₂O 0.5 g, CaCl₂·2H₂O 15 mg, ThiaminHCl 2 mg, and Glucose 0.2 g/mL, pH7.0) at 30°C for 24 hours. 100 µl of the culture solution was applied into the same medium (4 ml) and further cultivated at 42°C for 24 hours. The culture solution was diluted with physiological saline, spread on the M9 minimum plate, cultured at 42°C for 12 hours to obtain a single colony. The obtained colony was applied to the LB agar plate and the LB agar plate containing 50µg/ml of ampicillin, and cultivated at 30°C for 12 hours. After the recombination process was repeated twice, the strain, which acquired the sensitivity to ampicillin and grown only on the LB agar gel, was selected from the resultant products. Using the chromosome DNA of the obtained strain, in which recombination was repeated twice, as template and the primers having the nucleotide sequences in SEQ.NOs.1 and 2, the *pepD* fragment was amplified by PCR. It was confirmed that the amplified *pepD* gene was the *pepD* gene fragment (approximately 1.4Kbp) with the *Nru* I-*Hpa* I site deleted and the *pepD* gene of the strain was replaced by the deficient *pepD* gene. Therefore, the selected strain was verified to be the *pepD* deficient strain.

### (2) Obtaining isoaspartyldipeptidase gene (iadA gene) deficient strain

Based on sequence information (DDBJ/EMBL/GeneBank Accession No. AE000503) on isoaspartyldipeptidase gene (*iadA* gene) in Gene Data Bank, the primers (5'-CAAGGAGTTACCATGATTGA (SEQ ID NO: 3) and 5'-AACCGTTTAAGCCGTTTCAA (SEQ ID NO: 4)) were synthesized. The primers were used to amplify approximately 1.7 Kbp of the structural gene, which covered SD-ATG and the translation termination codon, by PCR (94°C, 1 min, 54°C, 2 min, 72°C, 3 min, 30 cycles) with the genome DNA of the *Escherichia coli* ATCC8739 strain as template. The gene was ligated to the *Sma* I site of the pUC18 vector using SureClone Ligation Kit to form pUC18-*iadA*. The competent cells of the *Escherichia coli* JM109 strain transformed with the plasmid were cultivated in the liquid medium containing 50µg/ml of ampicillin at 37°C for 16 hours and the plasmid was prepared from the recovered transformant. Then, pUC18-*iadA* was cleaved at the *Hpa* I site (the restriction enzyme site in 1.7 Kbp of the inserted fragment containing the idaA gene) and ligated to an *Eco*R I-*Not* I-*Bam*H I adapter (supplied from TaKaRa). The plasmid was further cleaved at the *Not* I site to induce the self-ligation reaction. Using the ligation reaction mixture, the competent cells of the *Escherichia coli* JM109 strain were transformed and the plasmid was prepared from the transformant grown in the LB agar plate containing 50µg/ml of ampicillin. From the resultant products, the plasmid DNA, pUCΔ*iadA* cleaved at the Not I site, was selected. It is expected that in the *iadA* gene contained in the pladmid DNA, frame shift occurs at the *Hpa* I site and the enzyme to be encoded has been inactivated .

Then, pUCΔ*iadA* was cleaved at the *Eco*R I and *Sal* I sites, 1.7 Kbp of DNA fragment containing the deficient *iadA* gene was prepared and ligated to the *Eco*R I/*Sal* I site of pMAN997, the vector for homologous replacement having the temperature-sensitivity replication origin (tsori) to construct the plasmid for the deficient *iadA* gene, pMANΔ*iadA*. The *Escherichia coli* JM 109 strain transformed with pMANΔ*iadA* was cultivated in the LB liquid medium containing 50µg/ml of ampicillin at 30°C for 16 hours and pMANΔ*iadA* was prepared from the recovered transformant. Using the plasmid for the deficient *iadA* gene, the *pepD* deficient strain of the aforementioned *Escherichia coli* ATCC8739 was transformed by electroporation. Subsequently, in the same manner, the *iadA* gene was replaced by the deficient *iadA* gene to obtain the strain, in which recombination was repeated twice and which has acquired the sensitivity to ampicillin. Using the chromosome DNA of the strain, in which recombination was repeated twice, as the template and the primers for the sequences categorized in SEQ.NOs. 3 and 4 the *iadA* fragment was amplified by PCR. It was confirmed that the amplified *iadA* fragment was cleaved at the restriction enzyme *Not* I site and the *iadA* gene of the strain was replaced by the deficient *iadA* gene. The obtained strain was verified to be a *pepD-iadA* double deficient strain.

### (3) Obtaining α-aspartyldipeptidase gene (pepE gene) deficient strain

Based on sequence information (DDBJ/EMBL/GeneBank Accession No. AE000475) on α-aspartyldipeptidase gene (*pepE* gene) in Gene Data Bank, the primers (5'-TATTTGTTATTT CCATTGGC (SEQ ID NO: 5) and 5'-AATGTCGCTCAACCTTGAAC (SEQ ID NO: 6)) were synthesized. The primers were used to amplify approximately 1.4 Kbp of structural gene, which covered SD-ATG and the translation termination codon, by the PCR (94°C, 1 min, 54°C, 2 min, 72°C, 3 min, 30 cycles) using of the genome DNA of the *Escherichia coli* ATCC8739 strain as template. The gene was ligated to the *Sma* I site of the pUC18 vector using SureClone Ligation Kit to construct pUC18-pepE. The competent cells of the *Escherichia coli* JM109 strain transformed with the plasmid was cultivated in the liquid medium containing 50µg/ml of ampicillin at 37°C for 16 hours and the plasmid was prepared from the recovered transformant. Then, pUC18-*pepE* was cleaved at the *Nru* I site (the restriction enzyme site in 1.4 Kbp of inserted fragment containing the *pepE* fragment) and ligated to the *Eco*R I-*Not* I-*Bam*H I adapter. The plasmid was further cleaved at the *Not* I site to induce the self-ligation reaction. Using the ligation reaction mixture, the *Escherichia coli* JM109 competence cells were transformed and the plasmid was prepared from the transformant grown in the LB agar plate containing 50µg/ml of ampicillin. From the resultant products, the plasmid DNA, pUCΔ*pepE* to be separated at the Not I site was selected. It is expected that in the *pepE* gene contained in the plasmid DNA, frame sift occurs and the enzyme to be encoded has been inactivated.

Then, pUCΔ*pepE* was separated at the *Eco*R I and *Sal* I sites and 1.4 Kbp of DNA fragment containing the deficient *pepE* gene was prepared. The DNA fragment was ligated to the *Eco*R I/*Sal* I site of pMAN997, the vector for homologous replacement having the temperature sensitivity replication origin (tsori) to construct the plasmid pMANΔ*pepE* for the deficient *pepE* gene. The *Escherichia coli* JM 109 strain transformed with pMANΔ*pepE* was cultivated in the LB liquid medium containing 50µg/ml of ampicillin at 30°C for 16 hours and pMANΔ*pepE* was prepared from the recovered transformant. Using the plasmid for the deficient *pepE* gene, the *pepD-iadA* double deficient strain of the aforementioned *Escherichia coli* ATCC8739 by electroporation was transformed. Subsequently, in the same manner, the *pepE* gene was replaced by the deficient *pepE* gene to obtain the strain having the sensitivity to ampicillin, in which recombination was repeated twice. Using the chromosome DNA of the strain, in which recombination was repeated twice, as the template and the primers having the sequences in SEQ.NOs.5 and 6, the *pepE* fragment was amplified by PCR. It was confirmed that the amplified *pepE* fragment was separated at the restriction enzyme *Not* I site and the *pepE* gene of the strain was replaced by the deficient *pepE* gene. The strain was verified to be the *pepD-iadA-pepE* triple deficient strain.

### (4) Obtaining the aminopeptidase gene (pepA gene) deficient strain

Based on sequence information (DDBJ/EMBL/GeneBank Accession No. AE000496) on the amino peptidase gene (*pepA* gene) in Gene Data Bank, the primers (5'-ACAGCGGACATGAGTTACGA (SEQ ID NO: 7) and 5'-CCCGCTAAATTATGCGGAAC (SEQ ID NO: 8))were synthesized. The primers were used to amplify approximately 1.9 Kbp of structural gene, which covered SD-ATG and the translation termination codon by the PCR method (94°C, 1 min, 54°C, 2 min, 72°C, 3 min, 30 cycles) using the genome DNA of the *Escherichia coli* ATCC8739 strain as template. The gene was ligated to the *Sma* I site of the pUC18 vector using SureClone Ligation Kit to construct pUC18-*pep*A. The competent cells of the *Escherichia coli* JM109 strain transformed with the plasmid was cultivated in the liquid medium containing 50µg/ml of ampicillin at 37°C for 16 hours and the plasmid was prepared from the recovered transformant. Then, pUC18-*pepA* was cleaved at the *Hpa* I site (the restriction enzyme site in 1.9 Kbp of inserted fragment containing the *pepA* fragment) to induce the self-ligation reaction. Using the ligation reaction mixture, the competent cells of the *Escherichia coli* JM109 strain were transformed and the plasmid was prepared from the transformant grown in the LB agar plate containing 50µg/ml of ampicillin. From the plasmid, the plasmid DNA, pUCΔ*pepA* to be cleaved at the *Hpa* I site was selected. It is expected that in *pepA* contained in the plasmid DNA, the *Hpa* I*-Hpa* I region was lost and the enzyme to be encoded has been inactivated. Then, pUCΔ*pepA* was cleaved at the *Sac* I and *Sph* I sites and 1.9 Kbp of DNA fragment containing the deficient *pepE* gene was prepared. The DNA fragment was ligated to the *Sac* I/*Sph* I site of pMAN997, the vector for homologous replacement having the temperature sensitivity replication origin (tsori) to construct the plasmid pMANΔ*pepA* for the deficient *pepA* gene. The *Escherichia coli* JM 109 strain transformed with pMANΔ*pepA* was cultivated in the LB liquid medium containing 50µg/ml of ampicillin and pMANΔ*pepA* was prepared from the recovered transformant. Using the plasmid for the deficient *pepA* gene, the aforementioned triple deficient *Escherichia coli* ATCC8739 strain with the *pepD, iadA*, and *pepE* genes deleted was transformed by electroporation. Subsequently, in the same manner, the *pepA* gene was replaced by the deficient *pepA* gene to obtain the strain having the sensitivity to ampicillin, in which recombination was repeated twice. Using the chromosome DNA of the strain, in which recombination was repeated twice, as the template and the primers having the sequences in SEQ.NOs. 7 and 8, the *pepA* fragment was amplified by PCR. It was confirmed that since the amplified *pepA* fragment was cleaved at the restriction enzyme *Mul* I site and 849 bp and 1039 bp of DNA fragments were formed, the *pepA* gene of the strain was replaced by the deficient *pepA* gene. The strain was verified to be the *pepD-iadA-pepE-pepA* quadruple deficient strain.

### Example 2 Determination of aspartylphenylalanine decomposition activity in each of peptidase gene deficient strains

The parent strain and each of gene deficient strains were applied in 4 ml of LB medium and shake-cultured at 30°C for 16 hours. Then, 4 ml of culture solution was centrifuged (2200×g 15 min) to obtain microorganism cell precipitates. 4 ml of physiological saline was added to the microorganism cell precipitates and centrifuged (2200×g 15 min) to obtain the washed microorganism cells. The washed microorganism cells were suspended in 1 ml of ultrasonic homogenated solution (20 mM MOPS-KOH buffer (pH7.0) containing 1 mM of DTT). The suspension was subject to the ultrasonic disruption (with the apparatus supplied from Bioruptor, 7.5 min) and centrifuged (12000×g 10 min) to obtain a cell-free extracted solution. Using the protein assay CBB solution (supplied from Nakarai Tesk), the proteins were quantified by the Bradford method. Bovine serum albumin (supplied from SIGMA) was used as the standard protein.

100 µl of cell-free extracted solution was added in 100 µl of substrate solution (100 mM of aspartylphenylalanine, 2mM of MnCl₂, 100 mM of MOPS-KOH buffer (pH7.0)) and the reaction was performed at 30°C for 2 hours. After the reaction, the reaction mixture was heated at 95°C for 5 minutes, and 5-fold diluted with distilled water. 10µl of diluted reaction mixture was analyzed using an amino acid analyzer (L-8500, supplied from Hitachi) and produced aspartic acid and phenylalanine were quantified.

Aspartylphenylalanine decomposition activities in the parent strain and each of gene deficient strains are shown in Table 1. Note that the amount of enzyme, which forms 1 µmol of Phe/minute at 30°C, was defined as one unit. In the parent strain, the strong aspartylphenylalanine decomposition activity was observed, while it reduced as each of genes was stepwise deleted. In the quadruple deficient strain with the *pepD, iadA, pepE*, and *pepA* genes deleted, the decomposition activity decreased down to approximately 1.6% of that of the parent strain.

**Table 1**

| Strains | AP decomposition activity (mU/mg-protein) | AP decomposition activity (relative activity: %) |
|---|---|---|
| Escherichia coli ATCC8739 (parent strain) | 425.8 | 100 |
| *pepD* deficient strain | 66.9 | 16 |
| pepD, iadA double-deficient strain | 32.4 | 7.6 |
| pepD, iadA, pepE triple-deficient strain | 15.2 | 3.6 |
| pepD, iadA, pepE, pepA quadruple-deficient strain | 6.8 | 1.6 |

### Example 3 Determination of alanylglutamine decomposition activity in each of peptidase gene deficient strains

The parent strain and each of gene deficient strains were applied in 3 ml of LB medium (Bacto tryptone 1.0%, Bacto yeast extract 1.0%, NaCl 1.0%, PH7.0) and shake-cultured at 37°C for 16 hours. Then, 1 ml of culture solution was centrifuged (2200xg 15 min) to obtain the microorganism cell precipitates. 1 ml of physiological saline was added to the microorganism cell precipitates, and then centrifugation was performed (2200×g 15 min) to obtain the washed microorganism cells. 0.1 ml of substrate solution (100 mM alanylglutamine, 100 mM boric acid-NaOH buffer (pH9.0)) was added to the washed microorganism cells, and the reaction was performed at 30°C for 6 hours.

Alanylglutamine in the reaction mixture was quantified by HPLC under the conditions described below to find the quantity of discomposed alanylglutamine. Column:Inertsil ODS-2 (4.6 x 250 mm, supplied from GL science), mobilizing layer: 5mM sodium 1-octanesulfonate:Methanol=10:1.5 (pH 2.1 with conc.H₃PO₄), flow rate of 1.0 ml/min, temperature of 40°C, detection at UV210 nm.

The alanylglutamine decomposition activities in the parent strain and each of gene deficient strains are shown in the table below. In the parent strain, after the 6-hour reaction, 100 mM of alanylglutamine was completely decomposed while in the *pepD* deficient strain, alanylglutamine decomposition activity significantly reduced. When the *pepE* and *pepA* genes were deleted, alanylglutamine decomposition activity further reduced.

**Table 2**

| Strains | alanylgultamine remained (mM) | alanylgultamine decomposition activity (relative activity:%) |
|---|---|---|
| Escherichia coli JM 109 (parent strain) | 0 | 100 |
| *pepD* deficient strain | 71.2 | 28.8 |
| pepD, pepE double-deficient strain | 79.8 | 20.2 |
| pepD, pepE, pepA triple-deficient strain | 81.0 | 19.0 |

### Reference 1

### Isolation of Gene for Peptide-synthesizing enzyme Derived from Empedobacter brevis

Hereinafter, isolation of a gene for peptide-synthesizing enzyme will be explained. *Empedobacter brevis* strain FERM BP-8113 was used as the microbe. For isolating the gene, *Escherichia coli* JM-109 was used as a host while pUC118 was used as a vector.

### (1) Construction of PCR Primer Based on Determined Internal Amino Acid Sequence

A mixed primer having the nucleotide sequences indicated in SEQ ID NO: 11 and SEQ ID NO: 12, respectively, was constructed based on the amino acid sequences (SEQ ID NO: 9 and 10) determined by the Edman's decomposition method detecting the digestion product of lysyl endopeptidase of a peptide-synthesizing enzyme derived from the *Empedobacter brevis* strain FERM BP-8113.

### (2) Preparation of Microbial Cells

*Empedobacter brevis* strain FERM BP-8113 was cultivated at 30°C for 24 hours on a CM2G agar medium (containing glucose 5 g/l, yeast extract 10 g/l, peptone 10 g/I, sodium chloride 5 g/l, and agar 20 g/l, pH 7.0). One loopful cells of the resulting microbial cells was inoculated into a 500 ml Sakaguchi flask containing 50 ml of a CM2G liquid medium (the aforementioned medium excluding agar) followed by shake culture at 30°C.

### (3) Preparation of Chromosomal DNA from Microbial Cells

50 ml of culture solution was centrifuged (12,000 rpm, 4°C, 15 minutes) to recover the microbial cells. Then, a chromosomal DNA was obtained from the microbial cells using the QIAGEN Genomic-Tip System (supplied from Qiagen) based on the procedure described in the manual therefor.

### (4) Preparation of DNA Fragment Containing Part of Gene for Peptide-synthesizing enzyme by PCR

A DNA fragment containing a portion of the gene for the peptide-synthesizing enzyme derived from *Empedobacter brevis* strain FERM BP-8113 was obtained by the PCR method using LA-Taq (supplied from Takara Shuzo). A PCR reaction was then carried out by using the primers having the nucleotide sequences of SEQ ID NOs: 11 and 12 to a chromosomal DNA obtained from *Empedobacter brevis* strain FERM BP-8113.

The PCR reaction was carried out for 30 cycles under the following conditions using the Takara PCR Thermal Cycler PERSONAL (supplied from Takara Shuzo).

| | |
|---|---|
| 94°C | 30 seconds |
| 52°C | 1 minute |
| 72°C | 1 minute |

After completion of the reaction, 3 µl of the racrion mixture was applied to 0.8% agarose electrophoresis. As a result, it was verified that a DNA fragment of about 1.5 kilobases (kb) was amplified.

### (5) Cloning of Gene for Peptide-synthesizing enzyme from Gene Library

In order to obtain the entire length of gene for peptide-synthesizing enzyme in full-length, southern hybridization was carried out by using the DNA fragment amplified in the PCR procedure as a probe. The procedure for southern hybridization is explained in Molecular Cloning, 2nd edition, Cold Spring Harbor Press (1989).

The approximately 1.5 kb DNA fragment amplified by the PCR procedure was separated by 0.8% agarose electrophoresis. The target band was then cut out and purified. The DNA fragment was labeled with digoxinigen as probe by using DIG High Prime (supplied from Boehringer-Mannheim) based on the procedure described in the manual therefor using DIG High Prime (supplied from Boehringer-Mannheim).

After completely digesting the chromosomal DNA of *Empedobacter brevis* obtained in the step (3) of the present Reference 1 by reacting at 37°C for 16 hours with restriction enzyme *Hin*dlll, the resultant was electrophoresed with on 0.8% agarose gel. The electrophoresed chromosomal DNA was blotted onto a positively charged Nylon membrane filter (supplied from Roche Diagnostics) from the agarose gel after the electrophoresis, followed by treatments consisting of alkaline denaturation, neutralization and immobilization. Hybridization was carried out by using EASY HYB (supplied from Boehringer-Mannheim). After pre-hybridizing the filter at 50°C for 1 hour, the probe labeled with digoxinigen prepared as described above was added and hybridization was carried out at 50°C for 16 hours. Subsequently, the filter was washed for 20 minutes at room temperature with 2 × SSC containing 0.1% SDS. Moreover, the filter was additionally washed twice at 65°C for 15 minutes with 0.1 × SSC containing 0.1% SDS.

Detection of bands that hybridized with the probe was carried out by using the DIG Nucleotide Detection Kit (supplied from Boehringer-Mannheim) based on the procedure described in the manual therefor using the DIG Nucleotide Detection Kit (supplied from Boehringer-Mannheim). As a result, a roughly 4 kb band was able to be detected that hybridized with the probe.

Then, 5 µg of the chromosomal DNA prepared in the step (3) of the present Reference 1 was completely digested with *Hin*dIII. A roughly 4 kb of DNA was separated by 0.8% agarose gel electrophoresis, followed by purification of the DNA using the Gene Clean II Kit (supplied from Funakoshi) and dissolving the DNA in 10 µl of TE. 4 µl of this product was then mixed with pUC118 *Hin*dIII/BAP (supplied from Takara Shuzo) and a ligation reaction was carried out by using the DNA Ligation Kit Ver. 2 (supplied from Takara Shuzo). 5 µl of the ligation reaction mixture and 100 µl of competent cells of *Escherichia coli* JM109 (supplied from Toyobo) were mixed to transform the *Escherichia coli*. This was then applied to a suitable solid medium to construct a chromosomal DNA library.

To obtain the full-length of gene for peptide-synthesizing enzyme, the chromosomal DNA library was screened by colony hybridization using the aforementioned probe. The procedure for colony hybridization is explained in Molecular Cloning, 2nd edition, Cold Spring Harbor Press (1989).

The colonies of the chromosomal DNA library were transferred on a Nylon membrane filter (Nylon Membrane for Colony and Plaque Hybridization, (supplied from Roche Diagnostics) followed by treatments consisting of alkali denaturation, neutralization and immobilization. Hybridization was carried out using EASY HYB (supplied from Boehringer-Mannheim). After pre-hybridizing the filter at 37°C for 1 hour, the aforementioned probe labeled with digoxinigen was added, followed by hybridization at 50°C for 16 hours. In addition, the filter was washed for 20 minutes at room temperature with 2 × SSC containing 0.1% SDS. Moreover, the filter was additionally washed twice at 65°C for 15 minutes with 0.1 × SSC containing 0.1% SDS.

Detection of colonies hybridizing with the labeled probe was carried out by using the DIG Nucleotide Detection Kit (supplied from Boehringer-Mannheim) based on the explanation described in the manual therefor using the DIG Nucleotide Detection Kit (supplied from Boehringer-Mannheim). As a result, two strains of colonies were verified to hybridize with the labeled probe.

### (6) Nucleotide sequence of Gene for Peptide-synthesizing enzyme Derived from Empedobacter brevis

Plasmids possessed by *Escherichia coli* JM109 were prepared from the aforementioned two strains of microbial cells that were verified to hybridize with the labeled probe by using the Wizard Plus Minipreps DNA Purification System (supplied from Promega) to and the nucleotide sequence of a portion where hybridization with the probe occurred and nearby was determined. The sequencing reaction was carried out by using the CEQ DTCS-Quick Start Kit (supplied from Beckman-Coulter) based on the procedure described in the manual therefor. In addition, electrophoresis was carried out by using the CEQ 2000-XL (supplied from Beckman-Coulter).

As a result, it was verified that an open reading frame that encodes a protein containing the internal amino acid sequences of the peptide-synthesizing enzyme (SEQ ID NOs: 9 and 10) did exist, thereby confirming that the open reading frame was a gene encoding the peptide-synthesizing enzyme. The nucleotide sequence of the full-length of the gene for peptide-synthesizing enzyme along with the corresponding amino acid sequence is shown in SEQ ID NO: 13. As a result of analysis on the homology of the resulting open reading frame with the BLASTP program, homology was discovered between the two enzymes; it showed with a homology of 34% as at the amino acid sequence level exhibited with the α-amino acid ester hydrolase of *Acetobacter pasteurianus* (see Appl. Environ. Microbiol., 68(1), 211-218 (2002), and a homology of 26% at the amino acid sequence level exhibited with the glutaryl-7ACA acylase of *Brevibacillus laterosporum* (see J. Bacteriol., 173(24), 7848-7855 (1991).

### (7) Expression of Gene for Peptide-synthesizing enzyme Derived from Empedobacter brevis in Escherichia coli

The promoter region of the trp operon on the chromosomal DNA of *Escherichia coli* W3110 was amplified by PCR using the oligonucleotides indicated in SEQ ID NOs: 15 and 16 as primers, and the resulting DNA fragments were ligated to a pGEM-Teasy vector (supplied from Promega). *E. coli* JM109 was then transformed with this ligation solution, and those strains having the target plasmid in which the direction of the inserted trp promoter is inserted in the opposite to the orientation from of the lac promoter were selected from ampicillin-resistant strains.

Next, a DNA fragment containing the trp promoter obtained by treating this plasmid with *Eco*O109I/*Eco*R I was ligated to an *Eco*O109I/*Eco*R I treatment product of pUC19 (supplied from Takara). *Escherichia coli* JM109 was then transformed with this ligation solution and those strains having the target plasmid were selected from ampicillin-resistant strains. Next, a DNA fragment obtained by treating this plasmid with *Hin*dIII/*Pvu*II was ligated with to a DNA fragment containing an rrnB terminator obtained by treating pKK223-3 (supplied from Amersham Pharmacia) with *Hind*III/*Hin*cII. *E. coli* JM109 was then transformed with this ligation solution, strains having the target plasmid were selected from ampicillin-resistant strains, and the plasmid was designated as pTrpT.

The target gene was amplified by PCR using the chromosomal DNA of *Empedobacter brevis* strain FERM BP-8113 as a template and the oligonucleotides indicated in SEQ ID NO: 17 and 18 as primers. This DNA fragment was then treated with *Nde*I/*Pst*I, and the resulting DNA fragment was ligated with the *Nde*I/*Pst*I treatment product of pTrpT. *Escherichia coli* JM109 was then transformed with this ligation solution, those strains having the target plasmid were selected from ampicillin-resistant strains, and this plasmid was designated as pTrpT_Gtg2.

*Escherichia coli* JM109 having pTrpT_Gtg2 was pre-cultured at 30°C for 24 hours in LB medium containing 100 mg/l of ampicillin. 1 ml of the resulting culture solution was transferred in a 500 ml Sakaguchi flask containing 50 ml of a medium (D-glucose 2 g/l, yeast extract 10 g/l, casamino acids 10 g/l, ammonium sulfate 5 g/l, potassium dihydrogen phosphate 3 g/l, dipotassium hydrogen phosphate 1 g/l, magnesium sulfate heptahydrate 0.5 g/l, and ampicillin 100 mg/l), followed by cultivation at 25°C for 24 hours. The culture solution had an α-L-aspartyl-phenylalanine-β-methyl ester forming activity of 0.11 U per 1 ml of culture solution and it was verified that the cloned gene was expressed by *E. coli*. Furthermore, no activity was detected for a transformant in which only pTrpT had been introduced as a control.

### Prediction of Signal Sequence

When the amino acid sequence of SEQ ID NO: 6 described in the Sequence Listing was analyzed with the Signal P v 1.1 program (see Protein Engineering, Vol. 12, No. 1, pp. 3-9, 1999), it was predicted that amino acids numbers 1 to 22 in amino acid sequence was operated as a signal that is secreted into the periplasm, while the mature protein was estimated to be downstream of amino acid number 23.

### Verification of Secretion

*Escherichia coli* JM109, having pTrpT_Gtg2, was pre-cultivated at 30°C for 24 hours in LB medium containing 100 mg/l of ampicillin. 1 ml of the resulting culture solution was transferred into a 500 ml Sakaguchi flask containing 50 ml of medium (glucose 2 g/l, yeast extract 10 g/l, casamino acids 10 g/l, ammonium sulfate 5 g/l, potassium dihydrogen phosphate 3 g/l, dipotassium hydrogen phosphate 1 g/l, magnesium sulfate heptahydrate 0.5 g/l, and ampicillin 100 mg/l), followed by mass-cultivation at 25°C for 24 hours to obtain cultivated microbial cells.

The cultivated microbial cells were fractionated into a periplasm fraction and a cytoplasm fraction by an osmotic pressure shock method using a 20 grams/deciliter (g/dl) sucrose solution. The microbial cells immersed in the 20 g/dl sucrose solution were immersed in a 5 mM aqueous MgSO₄ solution. The centrifuged supernatant was named a periplasm fraction ("Pe"). In addition, the centrifuged sediment was re-suspended and subjected to ultrasonic disrupting. The resultant was named a cytoplasm fraction ("Cy"). The activity of glucose 6-phosphate dehydrogenase, which is known to be present in the cytoplasm, was used as an indicator to verify that the cytoplasm had been separated. This measurement was carried out by adding a suitable amount of enzyme to a reaction mixture at 30°C containing 1 mM glucose 6-phosphate, 0.4 mM NADP, 10 mM MgSO₄, and 50 mM Tris-Cl (pH 8), followed by measurement of absorbance at 340 nm to measure production of NADPH.

The amounts of enzymes of in the periplasm fraction and the cytoplasm fraction when the activity of a separately prepared cell-free extract was assigned a value of 100% are measured. Glucose 6-phosphate dehydrogenase activity did not be observed in the periplasm fraction. It indicates that the periplasm fraction did not mix in the cytoplasm fraction. About 60% of the α-L-aspartyl-L-phenylalanine-β-methyl ester (α-AMP) synthesizing activity was recovered in the periplasm fraction, and it was verified that the Ala-Gln-forming enzyme was secreted into the periplasm as predicted from the amino acid sequence using the Signal P v 1.1 program.

### Reference 2

### Isolation of Gene for Peptide-synthesizing enzyme Derived from Sphingobacterium sp.

Hereinafter, isolation of a gene for peptide-synthesizing enzyme is described. The microbe used was *Sphingobacterium* sp. strain FERM BP-8124. For the isolation of the gene, *Escherichia coli* DH5α was used as a host, and pUC118 was used as a vector.

### (1) Preparation of Microbial Cells

*Sphingobacterium* sp. strain FERM BP-8124 was cultivated for 24 hours at 25°C on a CM2G agar medium (containing glucose at 50 g/l, yeast extract at 10 g/l, peptone at 10 g/l, sodium chloride at 5 g/l, and agar at 20 g/l, pH 7.0). One loopful cells of the resulting microbial cells was inoculated into a 500 ml Sakaguchi flask containing 50 ml of CM2G liquid medium (the aforementioned medium excluding agar) followed by shake culture at 25°C.

### (2) Preparation of Chromosomal DNA from Microbial Cells

50 ml of culture solution was centrifuged (12,000 rpm, 4°C, 15 minutes) to recover the microbial cells. A chromosomal DNA was then obtained from the microbial cells using the Qiagen Genomic-Tip System (supplied from Qiagen) based on the procedure described in the manual therefor.

### (3) Preparation of Probe DNA Fragment by PCR

A DNA fragment containing a gene for portion of the peptide-synthesizing enzyme derived from *Empedobacter brevis* strain FERM BP-8113 was obtained by the PCR method using LA-Taq (supplied from Takara Shuzo). A PCR reaction was then carried out by using primers having the nucleotide sequences of SEQ ID NOs: 11 and 12 to the chromosomal DNA obtained from *Empedobacter brevis* strain FERM BP-8113.

The PCR reaction was carried out by using the Takara PCR Thermal Cycler - PERSONAL (Takara Shuzo) for 30 cycles under the following conditions.

| | |
|---|---|
| 94°C | 30 seconds |
| 52°C | 1 minute |
| 72°C | 1 minute |

After completion of the reaction, 3 µl of reaction mixture was applied to 0.8% agarose electrophoresis. As a result, it was verified that a DNA fragment of about 1.5 kb was amplified.

### (4) Cloning of Gene for Peptide-synthesizing enzyme from Gene Library

In order to obtain the full-length gene for peptide-synthesizing enzyme, southern hybridization was carried out by using the DNA fragment amplified in the aforementioned PCR procedure as a probe. The procedure of southern hybridization is explained in Molecular Cloning, 2nd edition, Cold Spring Harbor Press (1989).

The approximately 1.5 kb DNA fragment amplified by the aforementioned PCR procedure was separated by 0.8% agarose electrophoresis. The target band was then cut out and purified. This DNA fragment was labeled with digoxinigen as probe by using DIG High Prime (supplied from Boehringer-Mannheim) based on the procedure described in the manual therefor.

After allowing the chromosomal DNA of *Sphingobacterium* sp. obtained in the step (2) of the present Reference 2 to react with restriction enzyme *Sac*I at 37°C for 16 hours to completely digest the DNA, the resultant was electrophoresed on 0.8% agarose gel. From the agarose gel after the electrophoresis, the electrophoresed chromosomal DNA was blotted onto a positively charged Nylon membrane filter (supplied from Roche Diagnostics), followed by treatments consisting of alkali denaturation, neutralization, and immobilization. Hybridization was carried out by using EASY HYB (supplied from Boehringer-Mannheim). After pre-hybridizing the filter at 37°C for 1 hour, the digoxinigen-labeled probe prepared as described above was added and hybridization was carried out at 37°C for 16 hours. Subsequently, the filter was washed twice at 60°C with 1 × SSC containing 0.1% SDS.

Detection of bands that hybridized with the probe was carried out by using the DIG Nucleotide Detection Kit (supplied from Boehringer-Mannheim) based on the procedure described in the manual therefor. As a result, a roughly 3 kb band was successfully detected that hybridized with the probe.

5 µg of the chromosomal DNA prepared in the step (2) of the present Reference 1 was completely digested with *Sac*I. About 3 kb of a DNA was separated by 0.8% agarose gel electrophoresis, the DNA was purified using the Gene Clean II Kit (supplied from Funakoshi), and dissolved in 10 µl of TE. 4 µl of the resulting solution and pUC118 treated with alkaline phosphatase (*E. coli* C75) at 37°C for 30 minutes and at 50°C for 30 minutes, after reaction with *Sac*I at 37°C for 16 hours to completely digest, were mixed and a ligation reaction was carried out by using the DNA Ligation Kit Ver. 2 (supplied from Takara Shuzo). 5 µl of this ligation reaction mixture and 100 µl of competent cells of *Escherichia coli* DH5α (supplied from Takara Shuzo) were mixed to transform the *Escherichia coli*. This was then applied to a suitable solid medium to produce a chromosomal DNA library.

To obtain full-length gene for peptide-synthesizing enzyme, the chromosomal DNA library was screened by colony hybridization using the aforementioned probe. The procedure for colony hybridization is explained in Molecular Cloning, 2nd edition, Cold Spring Harbor Press (1989).

The colonies of the chromosomal DNA library were transferred on a Nylon membrane filter (Nylon Membrane for Colony and Plaque Hybridization, supplied from Roche Diagnostics), followed by treatments of alkali denaturation, neutralization, and immobilization. Hybridization was carried out by using EASY HYB (supplied from Boehringer-Mannheim). After pre-hybridizing the filter at 37°C for 1 hour, the aforementioned digoxinigen-labeled probe was added, followed by hybridization at 37°C for 16 hours. Subsequently, the filter was washed twice at 60°C with 1 × SSC containing 0.1% SDS.

Detection of colonies hybridizing with the labeled probe was carried out by using the DIG Nucleotide Detection Kit (supplied from Boehringer-Mannheim) based on the explanation described in the manual therefor. As a result, six strains of colonies were verified to have hybridized with the labeled probe.

### (5) Nucleotide sequence of Gene for Peptide-synthesizing enzyme Derived from Sphingobacterium sp.

Plasmids possessed by *Escherichia coli* DH5α were prepared from the six strains of microbial cells that were verified to have hybridized with the labeled probe by using the Wizard Plus Minipreps DNA Purification System (supplied from Promega) to determine the nucleotide sequence of a portion where hybridization with the probe occurred and nearby was determined. The sequencing reaction was carried out by using the CEQ DTCS-Quick Start Kit (supplied from Beckman-Coulter) based on the procedure described in the manual therefor. In addition, electrophoresis was carried out by using the CEQ 2000-XL (supplied from Beckman-Coulter).

As a result, it revealed that an open reading frame that encodes peptide-synthesizing enzyme did exist. The full-length nucleotide sequence of the gene for peptide-synthesizing enzyme derived from *Sphingobacterium* sp. along with the corresponding amino acid sequence is shown in SEQ ID NO: 19. Peptide-synthesizing enzyme derived from *Sphingobacterium* sp. exhibited a homology of 63.5% at the amino acid sequence level to the peptide-synthesizing enzyme derived from *Empedobacter brevis* (as determined using the BLASTP program).

### (6) Expression of Gene for Peptide-synthesizing Enzyme Derived from Sphingobacterium sp. in Escherichia coli

The target gene was amplified by PCR using the chromosomal DNA of *Sphingobacterium* sp. FERM BP-8124 as a template and the oligonucleotides shown in SEQ ID NOs: 21 and 22 as primers. This DNA fragment was treated with *Nde*I/*Xba*I, and the resulting DNA fragment and an *Nde*I/*Xba*I treatment product of pTrpT were ligated. *Escherichia coli* JM109 was then transformed with this ligation solution, and strains having the target plasmid were selected from ampicillin-resistant strains. The plasmid was designated as pTrpT_Sm_aet.

*Escherichia coli* JM109 having pTrpT_Sm_aet was cultivated at 25°C for 20 hours by inoculating one loopful cells thereof into an ordinary test tube containing 3 ml of a medium (glucose 2 g/l, yeast extract 10 g/l, casamino acids 10 g/l, ammonium sulfate 5 g/l, potassium dihydrogen phosphate 3 g/l, dipotassium hydrogen phosphate 1 g/l, magnesium sulfate heptahydrate 0.5 g/l and ampicillin 100 mg/l). It was verified that a cloned gene having an α-AMP production activity of 0.53 U per ml of culture solution was expressed by *Escherichia coli*. Furthermore, no activity was detected for a transformant containing only pTrpT used as a control.

### Prediction of Signal Sequence

When the amino acid sequence of SEQ ID NO: 20 described in the Sequence Listing was analyzed with the Signal P v 1.1 program (Protein Engineering, Vol. 12, No. 1, pp. 3-9, 1999), it was predicted that amino acids numbers 1 to 20 function as a signal that is secreted into the periplasm, while the mature protein was estimated to be downstream of amino acid number 21.

### Confirmation of Signal Sequence

One loopful cells of *Escherichia coli* JM109, having pTrpT_Sm_aet, was inoculated into ordinary test tubes containing 50 ml of a medium (glucose 2 g/l, yeast extract 10 g/l, casamino acids 10 g/l, ammonium sulfate 5 g/l, potassium dihydrogen phosphate 3 g/l, dipotassium hydrogen phosphate at 1 g/l, magnesium sulfate heptahydrate 0.5 g/l and ampicillin 100 mg/l) and main cultivation was performed at 25°C for 20 hours.

Hereinafter, procedures after centrifugal separation were carried out either on ice or at 4°C. After the cultivation, the microbial cells were separated from the culture solution by centrifugation, washed with 100 mM phosphate buffer (pH 7), and then suspended in the same buffer. The microbial cells were then subjected to ultrasonic disrupting treatment for 20 minutes at 195 W, the ultrasonic disrupted solution was centrifuged (12,000 rpm, 30 minutes) to remove the debris and obtain a soluble fraction. The resulting soluble fraction was applied to a CHT-II column supplied from Biorad) pre-equilibrated with 100 mM phosphate buffer (pH 7), and enzyme was eluted at a linear concentration gradient with 500 mM phosphate buffer. A solution obtained by mixing the active fraction with 5-fold volumes of 2 M ammonium sulfate and 100 mM phosphate buffer was applied to a Resource-PHE column (supplied from Amersham) pre-equilibrated with 2 M ammonium sulfate and 100 mM phosphate buffer, and an enzyme was eluted at a linear concentration gradient by 2 to 0 M ammonium sulfate to obtain an active fraction solution. As a result of these procedures, it was verified that the peptide-synthesizing enzyme was electrophoretically uniformly purified.

When the amino acid sequence of the aforementioned peptide-synthesizing enzyme was determined by Edman's decomposition method, the amino acid sequence of SEQ ID NO: 15 was obtained, and the mature protein was verified to be downstream of amino acid number 21 as was predicted by the SignalP v 1.1 program.

### Example 4 Production of alanylglutamine by the strain derived from Empedobacter aerogenes with amino acid ester transpeptidase highly expressed in the host, each of peptidase deficient strains

The plasmid pTrpT-aet derived from *Empedobacter brevis* for expressing amino-acid ester transpeptidase, which was constructed in the aforementioned Reference 1, was introduced by electroporation into the *Escherichia coli* JM109 strain, and the *pepD-pepE* double deficient strain and *pepD-pepE-pepA* triple deficient strain produced in Example 1. Each of strains was cultivated in the L-agar medium containing ampicillin (100µg/mL)at 20°C for 48 hours. Then, a 1/4-plate allot of cells were transferred in 50 ml of L-medium (10g/L of peptone, 5g/L of yeast extract, 10g/L of NaCl). Using a shaker (140rpm), 3 ml of culture solution (OD₆₁₀=0.15, 26-fold diluted), which was cultivated at 22°C for 16 hours, was applied to 300 ml of medium with the composition shown below, followed by batch-cultivating in an experimental fermenter with 1.0 L of capacity while staring air flow (1/1vvm)at 700 rpm. 15 ml of culture solution after sugar lost (OD₆₁₀=0.60, 51-fold diluted) was applied into 300 ml of medium with the same composition and cultivated in the same fermenter at 20°C while staring air flow (1/1vvm)and feeding sugar. The pH value was automatically adjusted to 7.0 with gaseous ammonium.

### Composition of medium (g/L):

Glucose 25.0
MgSO₄ · 7H₂O 1.0
(NH₄)₂SO₄ 5.0
H₃PO₄ 3.5
FeSO₄ · 7H₂O 0.05
MnSO₄ · 5H₂O 0.05
Mameno (TN) 0.45
GD113 0.1
ampicillin 0.1

Glucose and magnesium sulfate were separately sterilized. The pH values for other components were adjusted to 5.0 by KOH.
Composition of feeding liquid sugar(g/L)
Glucose 500.0
pH not-adjusted

2 ml of culture solution was centrifuged (2200xg15 min) to obtain the microorganism precipitates. 2 ml of physiological saline was added to the microorganism cell precipitates, followed by centrifugation (2200xg 15 min) to obtain the washed microorganism cells. The washed microorganism cells was added into 10 ml of reaction mixture containing 100 mM of L-alanine-methyl ester hydrochloride (14.0 mg/ml), 200 mM of L-glutamine(14.6 mg/ml)(0.2M), 100 mM of boric acid-NaOH buffer (pH9.0) and the alanylglutamine production reaction was induced under the conditions, pH 9.0 and 25°C. The result is shown in FIG.1.

The initial velocity of the reaction was almost the same. However, in the reaction using the microorganisms, for which the host was the *Escherichia coli* JM109 strain, alanylglutamine was further dissolved as the reaction time was extended. In the reaction using the *pepD-pepE* double deficient strain and the *pepD-pepE-pepA* triple deficient strain as the hosts, even through the reaction time was extended, the decomposition of formed alanylglutamine was suppressed. In the actual production reaction, it is difficult to check the termination point, at which the reaction rapidly stops. Accordingly, it might be preferable to use the *pepD-pepE* double deficient strain or *pepD-pepE-pepA* triple deficient strain as the hosts for alanylglutamine production.

Although the invention has been described with respect to a specific embodiment for a complete and clear disclosure, the appended claims are not to be thus limited but are to be construed as embodying all modifications and alternative constructions that may occur to one skilled in the art which fairly fall within the basic teaching herein set forth.

### SEQUENCE FREE TEXT

SEQ ID NO: 1; PCR primer
SEQ ID NO: 2; PCR primer
SEQ ID NO: 3; PCR primer
SEQ ID NO: 4; PCR primer
SEQ ID NO: 5; PCR primer
SEQ ID NO: 6; PCR primer
SEQ ID NO: 7; PCR primer
SEQ ID NO: 8; PCR primer
SEQ ID NO: 9; amino acid sequence of enzyme determined by Edman degradation derived from *Embedobacter brevis*
SEQ ID NO: 10; amino acid sequence of enzyme determined by Edman degradation derived from *Embedobacter brevis*
SEQ ID NO: 11; mix primer
SEQ ID NO: 12; mix primer
SEQ ID NO: 13; peptide-synthesizing enzyme derived from *Embedobacter brevis*
SEQ ID NO: 14; peptide-synthesizing enzyme derived from *Embedobacter brevis*
SEQ ID NO: 15; primer for pTrpT preparation
SEQ ID NO: 16; primer for pTrpT preparation
SEQ ID NO: 17; primer for pTrpT_Gtg2 preparation
SEQ ID NO: 18; primer for pTrpT_Gtg2 preparation
SEQ ID NO: 19; peptide-synthesizing enzyme derived from *Sphingobacterium SP*
SEQ ID NO: 20; peptide-synthesizing enzyme derived from *Sphingobacterium SP*
SEQ ID NO: 21; primer for pTrpT_Sm_aet preparation
SEQ ID NO: 22; primer for pTrpT_Sm_aet preparation

## Claims

1. A mutant microorganism of which at least one gene is disrupted,
wherein the gene is selected from the group consisting of a gene encoding aminoacylhistidine peptidase, a gene encoding leucylaminopeptidase and a gene encoding isoaspartyldipeptidase, respectively on a chromosome.

2. The microorganism according to claim 1, of which a gene encoding α-aspartyldipeptidase on the chromosome is disrupted.

3. The microorganism according to claim 1 or 2, which is a bacterium belonging to the genus *Escherichia.*

4. A mutant microorganism:
of which at least one gene is disrupted, wherein the gene is selected from the group consisting of a gene encoding aminoacylhistidine peptidase, a gene encoding leucylaminopeptidase and a gene encoding isoaspartyldipeptidase, respectively on a chromosome; and
which is transformed with a recombinant DNA including a polynucleotide encoding a protein having peptide-synthesizing activity.

5. The microorganism according to claim 4, of which a gene encoding α-aspartyldipeptidase on the chromosome is disrupted.

6. The microorganism according to claim 4 or 5, which is a bacterium belonging to a genus *Escherichia*.

7. The microorganism according to claim 4 or 5, wherein the protein having peptide-synthesizing activity is derived from the bacterium belonging to the genus *Empedobacter* or *Sphingobacterium*.

8. The microorganism according to claim 4 or 5, wherein the protein having the peptide-synthesizing activity is selected from the group consisting of (A) and (B):
(A) a protein comprising an amino acid sequence of SEQ ID NO: 14,
(B) a protein comprising an amino acid sequence including substitution, deletion, insertion, addition, and/or inversion of one or several amino acid residues in the amino acid sequence of SEQ ID NO: 14, and having the peptide-synthesizing activity.

9. The microorganism according to claim 4 or 5, wherein the protein having the peptide-synthesizing activity is selected from the group consisting of (C) or (D):
(C) a protein comprising an amino acid sequence of SEQ ID NO: 20,
(D) a protein comprising an amino acid sequence including substitution, deletion, insertion, addition, and/or inversion of one or several amino acid residues in the amino acid sequence of SEQ ID NO: 20, and having the peptide-synthesizing activity.

10. A method for producing a microorganism comprising:
disrupting at least one gene selected from the group consisting of a gene encoding aminoacylhistidine peptidase, a gene encoding leucylaminopeptidase and a gene encoding isoaspartyldipeptidase, respectively on a chromosome.

11. The method for producing a microorganism according to claim 10, comprising:
disrupting a gene encoding α-aspartyldipeptidase on the chromosome.

12. A method for producing a microorganism comprising:
disrupting at least one gene selected from the group consisting of a gene encoding aminoacylhistidine peptidase, a gene encoding leucylaminopeptidase and a gene encoding isoaspartyldipeptidase, respectively on a chromosome; and
transforming with a recombinant DNA including a polynucleotide encoding a protein having peptide-synthesizing activity.

13. The method for producing a microorganism according to claim 12, comprising:
disrupting a gene encoding α-aspartyldipeptidase on the chromosome.

14. A method for producing a peptide comprising:
cultivating in medium a microorganism of which at least one gene is disrupted, wherein the gene is selected from the group consisting of a gene encoding aminoacylhistidine peptidase, a gene encoding leucylaminopeptidase and a gene encoding isoaspartyldipeptidase, respectively on a chromosome, and wherein the microorganism is transformed with a recombinant DNA including a polynucleotide encoding a protein having peptide-synthesizing activity; and
mixing at least one of the microorganism cultivated and a disrupted cell of the microorganism with a carboxy component and an amine component to form a peptide.

15. The method for producing a peptide according to claim 14,
wherein a gene encoding α-aspartyldipeptidase on a chromosome of the microorganism is disrupted.
